# EUROPEAN PATENT SPECIFICATION

(11) **EP 4 359 076 B1**
(45) Date of publication and mention of the grant of the patent: **06.08.2025**
(21) Application number: 22737436.0
(22) Date of filing: 21.06.2022
(51) Int. Cl.: A61K 9/28, A61K 9/20, A61K 9/00, A61P 9/10, A61P 11/00, A61P 17/00, A61P 17/06, A61P 19/02, A61P 29/00, A61P 31/12, A61P 37/06, C07C 51/00, C07C 233/83, C07C 309/29, C07D 207/42, A61K 31/402, A61P 43/00, A61P 35/00, A61P 35/02

(54) **PHENYL PYRROLE AMINOGUANIDINE SALTS AND FORMULATIONS**
PHENYLPYRROLAMINOGUANIDINSALZE UND FORMULIERUNGEN
SELS DE PHÉNYL PYRROLE AMINOGUANIDINE ET FORMULATIONS ASSOCIÉES

(30) Priority: 21.06.2021 EP 21180702; 21.06.2021 EP 21180708; 23.11.2021 EP 21209855
(43) Date of publication of application: 01.05.2024
(73) Proprietor: SynAct Pharma ApS, 2840 Holte (DK)
(72) Inventor: BOESEN, Thomas, 2100 Copenhagen Ø (DK); JONASSEN, Thomas Engelbrecht Nordkild, 2840 Holte (DK); HØJGAARD, Bent, 3450 Allerød (DK)
(74) Representative: Høiberg P/S
(86) International application number: PCT/EP2022/066906
(87) International publication number: WO 2022/268825

(56) References cited:
- WO-A1-2019/243625
- WO-A1-2020/229297
- WO-A1-2021/198223

## Description

### Technical field

The present invention relates to pharmaceutically acceptable salts of AP1189 ((*E*)*-N-*[1-(2-nitrophenyl)-1-*H*-pyrrole-2-yl-allylideneamino]-guanidine) which are highly soluble at low pH, such as having a solubility of more than 10 mM at pH 1.2, pharmaceutical compositions comprising same, and solid oral formulations comprising a pharmaceutically acceptable salt of AP1189.

### Background

Melanocortin (MC) receptors (MC1R-MC5R), a family of class A G protein-coupled receptors (GPCRs), are attractive therapeutic targets for a number of conditions due to their wide distribution and diversity of physiological processes they regulate. MC1R regulates UV light-induced skin tanning and other immune responses because of its expression on leukocytes. MC2R regulates cortisol production on the adrenal glands, whereas MC5R plays a role on exocrine glands secretions. MC3R and MC4R exert non-redundant functions on energy homeostasis in addition to specific anti-inflammatory roles; whereas MC3R activation is particularly protective for joint inflammation such as arthritis, MC4R provides neuroprotection in brain inflammation. Accordingly, an array of pathological situations could be targeted with MCR-drugs including skin conditions, cardiovascular pathologies, joint inflammation, obesity and cachexia.

Peripheral MC1R and MC3R can be pharmacologically activated to induce antiinflammation. The endogenous agonist α-melanocyte-stimulating hormone (αMSH), like other protective mediators, is released by immune cells to counterbalance proinflammatory signals, thus preventing excessive tissue damage. In line with the resolution of inflammation concept, therapeutics targeting MC1R and MC3R act by mimicking the body's own protective resources and might be characterized by a lighter burden of side effects.

Shown to be effective in rheumatic diseases since the early 1950s, the use of corticotropin or adrenocorticotropin hormone (ACTH) declined when synthetic glucocorticoids became available. However, the discovery of an alternative anti-inflammatory mechanism for ACTH involving activation of peripheral MC receptors on immune cells has revived the interest in developing novel ACTH-like molecules with no steroidogenic effects for the treatment of joint diseases such as gout or RA (rheumatoid arthritis). The limitation in the translational delivery of novel MC drugs besides the marketed ACTH formulations is imposed by the lack of receptor selectivity achieved so far.

Innovative approaches in G protein-coupled receptor drug discovery might help to overcome this limitation. Allosteric modulation consists in the ability of a molecule to enhance (positive modulation) or reduce (negative modulation) the effect of the endogenous ligand by binding to a distinct site of the receptor protein, termed allosteric site. A higher degree of selectivity is expected as allosteric regions are less conserved among the five MCRs, and indeed, allosteric modulators at MC4R are currently under development for the treatment of obesity.

Another emerging concept of significant therapeutic interest is the one of biased agonism. The obsolete notion that receptors could exist in two unique conformations, the active one and the inactive one, has been replaced with the conception that multiple active conformations can exist, each one creating a distinct signal yielding multiple functional outcomes. Receptor activation, rather than linear and static, is emerging as a highly dynamic and multidimensional process in which a diversity of active conformations may be induced by different molecules leading to distinct effects.

Melanocortin therapy by using adrenocorticotropic hormone (ACTH) or non-steroidogenic melanocortin peptides attenuates proteinuria and glomerular injury in experimental glomerular diseases and induces remission of nephrotic syndrome in patients with diverse glomerulopathies, even those resistant to steroids.

Phenyl pyrrole aminoguanidine compounds with activity on MCRs are disclosed in WO 2007/141343.

The small molecule AP1189 ((E)-N-trans-{3-[1-(2-nitrophenyl)-1H-pyrrol-2-yl]-allylidene}-aminoguanidine) (or specifically N"-[(E)-[(2E)-3-[1-(2-nitrophenyl)-1H-pyrrol-2-yl]prop-2-en-1-ylidene]amino]guanidine) has been characterized as a biased agonist at receptors MC1R and MC3R, which does not induce canonical cAMP generation, but cause ERK1/2 phosphorylation, a signalling responsible for the proefferocytic effect evoked in primary macrophages. AP1189 was shown to reduce cytokine release in macrophages, whereas no melanogenesis was induced by AP1189 in melanocytes. In vivo, AP1189 has been shown to elicit anti-inflammatory actions in peritonitis and to accelerate the resolution phase, and to significantly reduce macroscopic and histological parameters of joint disruption in experimental inflammatory arthritis. AP1189 is thus a biased dual agonist at MC1R and MC3R with anti-inflammatory properties together with a lack of effect on melanogenesis.

Drug absorption in the stomach is usually a minor player in the total absorption of a drug dose, because the stomach has a smaller surface area, and the drug usually does not spend very long in there. Moreover some drugs are ionised by gastric pH and do not absorb very well. Some drugs are degraded by gastric pH, which reduces their absorption. Gastric pH and rate of stomach emptying can thus influence the availability and exposure of all oral drugs.

AP1189 is currently investigated in clinical trials for its efficacy in the kidney disease iMN (idiopathic membranous nephropathy), in rheumatoid arthritis in combination with methotrexate (MTX), as well as in acute respiratory distress syndrome (ARDS) in COVID19-patients. The formulations currently employed in the clinic are suspensions comprising an alkaline excipient, which followed an earlier enteric coated tablet formulation (targeting the small intestine). The enteric coated tablet demonstrated suboptimal absorption and low bioavailability.

WO 2020/229297 discloses Hippurate, besylate, oxoglutarate, glutarate, adipate, and nitrate salts of AP1189.

### Summary

In order to circumvent degradation at low pH, including low pH of the gastric compartment, AP1189 has been formulated as an enteric coated tablet and later as an alkaline suspension. It is now demonstrated that a subset of the pharmaceutically acceptable salts of AP1189 are highly soluble at low pH, including at pH 1.2 mimicking gastric pH. For example, the acetate and the succinate salt forms of AP1189 are surprisingly highly soluble at low pH, whereas other salt forms do not demonstrate such high solubility at low pH (e.g. tosylate salt forms of AP1189).

Therefore, based on this finding, it has now been demonstrated that certain pharmaceutically acceptable salts of AP1189, including AP1189 in its acetate salt and succinate salt forms, do not require protection from low pH, including the low pH of the gastric compartment, such as has been attempted in the previous enteric coated tablets and in the current alkaline suspensions. On the contrary, it is now demonstrated that an immediate release solid oral formulation has superior bioavailability compared to enteric coated tablets and is superior or comparable to alkaline suspensions of AP1189, and its pharmaceutically acceptable salts, which salts are highly soluble at low pH. A high solubility at low pH, such as at pH 1.2, facilitates fast release and absorption in the gastric compartment after oral ingestion.

Formulating AP1189 and its pharmaceutically acceptable salts in a solid oral formulation targeting the gastric compartment is superior to the currently available formulations in terms of improving bioavailability and exposure, and providing ease of administration.

One aspect of the present disclosure provides an immediate release solid oral dosage form comprising a pharmaceutically acceptable salt of AP1189 ((*E*)-*N*-[1-(2-nitrophenyl)-1-*H*-pyrrole-2-yl-allylideneamino]-guanidine) of formula I: 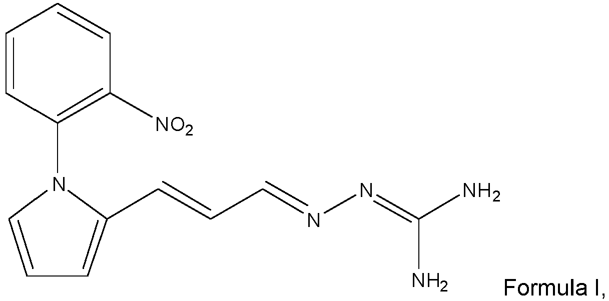 including tautomeric and stereoisomeric forms thereof, wherein said pharmaceutically acceptable salt is selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and further comprising at least one pharmaceutically acceptable excipient selected from the group consisting of a binder, a disintegrant and a wetting agent.

It is an aspect of the present disclosure to provide a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, wherein said pharmaceutically acceptable salt has high solubility at low pH.

In some embodiments the pharmaceutically acceptable salt of AP1189 has high solubility at gastric pH, such as at a pH of 0.5 to 3.5, such as at a pH of 1.2.

In some embodiments the solubility of said pharmaceutically acceptable salt of AP1189 is at least 10 mM at pH 1.2, such as at least 12 mM at pH 1.2, such as at least 15 mM at pH 1.2, such as at least 20 mM at pH 1.2, or more. In some embodiments the solubility of said pharmaceutically acceptable salt of AP1189 is at least 10 mM at pH 1.2. In some embodiments the solubility of said pharmaceutically acceptable salt of AP1189 is at least 15 mM at pH 1.2. In some embodiments the solubility of said pharmaceutically acceptable salt of AP1189 is at least 50 mM at pH 1.2.

In some embodiments the pharmaceutically acceptable salt of AP1189 is selected from the group consisting of:
the acetate salt of AP1189 ((*E*)-*N*-[1-(2-nitrophenyl)-1-*H*-pyrrole-2-yl-allylideneamino]-guanidinium acetate; 'AP1189 acetate'), and
the succinate salt of AP1189 ((E)-*N*-[1-(2-nitrophenyl)-1-*H*-pyrrole-2-yl-allylideneamino]-guanidinium succinate; 'AP1189 succinate').

In some embodiments the pharmaceutically acceptable salt of AP1189 is selected from the acetate salt of AP1189 ((*E*)-*N*-[1-(2-nitrophenyl)-1-*H*-pyrrole-2-yl-allylideneamino]-guanidinium acetate), and the succinate salt of AP1189 ((*E*)-*N*-[1-(2-nitrophenyl)-1-*H-*pyrrole-2-yl-allylideneamino]-guanidinium succinate).

It is also an aspect of the present disclosure to provide an oral formulation comprising a pharmaceutically acceptable salt of AP1189 as disclosed herein. In some embodiments said oral formulation delivers the pharmaceutically acceptable salt of AP1189 primarily to the gastric compartment, and enables gastric absorption of said AP1189.

In some embodiments the oral formulation comprising a pharmaceutically acceptable salt of AP1189 is a solid oral dosage form. In some embodiments the oral formulation comprising a pharmaceutically acceptable salt of AP1189 is an immediate release solid oral dosage form.

It is also an aspect of the present disclosure to provide an oral formulation comprising a pharmaceutically acceptable salt of AP1189 for use in the treatment of a disease or disorder, including a kidney disease, an arthritic disease, a viral disease or disorder, a cardiovascular disease and/or atherosclerosis, and systemic inflammatory disorders.

The invention is defined by the claims. Any subject matter falling outside the scope of the claims is provided for information purposes only.

### Description of Drawings

Figure 1: Mean AP1189 plasma concentration vs. time by treatment. Treatment A (squares): AP1189 acetate powder in suspension. Treatment B (triangles): AP1189 acetate enteric coated tablet formulation, fasting. Treatment C (circles): AP1189 acetate enteric coated tablet formulation, fed. See Example 5.
Figure 2: Mean AP1189 plasma concentrations (ng/mL) vs. time. Day 1 (circles). Day 7 (triangles). Day 14 (squares) (enteric coated tablets). See Example 6.
Figure 3: AUC₀₋₂₄ (plasma concentration of AP1189) shown against formulation (AP1189 acetate oral suspension, alkaline; AP1189 acetate immediate release tablet 'AP1189', and AP1189 succinate immediate release tablet 'SP1189'). Data was pooled for Day 1 and 5. See Example 8.
Figure 4: Cₘₐₓ (plasma concentration of AP1189) shown against formulation (AP1189 acetate oral suspension, alkaline; AP1189 acetate immediate release tablet 'AP1189', and AP1189 succinate immediate release tablet 'SP1189'). Data was pooled for Day 1 and 5. See Example 8.
Figure 5: Plasma concentration of AP1189 shown against time 0 - 24 hours. Data shown for AP1189 acetate oral suspension, alkaline; AP1189 acetate immediate release tablet 'AP1189', and AP1189 succinate immediate release tablet 'SP1189'. See Example 9.

### Definitions

The term "pharmaceutically acceptable derivative" in the present context includes pharmaceutically acceptable salts, which indicate a salt which is not harmful to the subjects. Such salts include pharmaceutically acceptable basic or acid addition salts as well as pharmaceutically acceptable metal salts, ammonium salts and alkylated ammonium salts. A pharmaceutically acceptable derivative further includes crystal forms of a compound.

A pharmaceutically acceptable salt of AP1189 is considered as highly soluble at low pH according to the present disclosure when the solubility of the pharmaceutically acceptable salt of AP1189 is at least 10 mM at pH 1.2, such as at least 12 mM at pH 1.2, such as at least 15 mM at pH 1.2, such as at least 20 mM at pH 1.2.

Reference to a pharmaceutically acceptable salt of AP1189 ((*E*)-*N*-[1-(2-nitrophenyl)-1-*H*-pyrrole-2-yl-allylideneamino]-guanidine) including tautomeric and stereoisomeric forms thereof, is meant to encompass amorphous forms and well as any polymorphic (crystalline) forms of such salts. The skilled person knows how to prepare polymorphic forms of AP1189 salts. Polymorphic forms of pharmaceutically acceptable salts of AP1189 are disclosed in WO 2022/268814.

The term "pharmaceutically effective amount" of a compound as used herein refers to an amount sufficient to cure, alleviate, prevent, reduce the risk of, or partially arrest the clinical manifestations of a given disease or disorder and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The terms "treatment" and "treating" as used herein refer to the management and care of a subject for the purpose of combating a condition, disease or disorder. The term is intended to include the full spectrum of treatments for a given condition from which the subject is suffering. The subject to be treated is preferably a mammal, in particular a human being. Treatment of animals, such as mice, rats, dogs, cats, horses, cows, sheep and pigs, is, however, also within the scope of the present context. The subjects to be treated can be of various ages.

An "arthritic disease" as referred to herein is an inflammatory disease which presents with joint inflammation. Also known as arthritis.

pH is the measurement of the acidity or alkalinity of a solution, or a negative logarithmic scale of hydrogen ion concentration in a solution. The greater the hydrogen ion concentration, the more acidic the solution, which is expressed as a lower pH. The lower the hydrogen ion concentration, the more alkaline the solution, which is expressed as a higher pH. The pH scale goes from 1 (strongly acidic) through 7 (neutral), to 14 (strongly alkaline).

The terms "approximately" and "about" as referred herein are synonymous. In some embodiments, "approximately" and "about" refer to the recited amount, value, or duration ± 5%, ± 4.5%, ± 4%, ±3.5%, ±3%, ±2.5%, ±2%, ±1.75%, ±1.5%, ±1.25%, ±1%, ±0.9%, ±0.8%, ±0.7%, ±0.6%, ±0.5% ±0.4%, ±0.3%, ±0.2%, ±0.1%, ±0.09%, ±0.08%, ±0.07%, ±0.06%, ±0.05%, ±0.04%, ±0.03%, ±0.02%, or ±0.01%. In some embodiments, "approximately" and "about" refer to the listed amount, value, or duration ±2.5%, ±2%, ±1.75%, ±1.5%, ±1.25%, ±1%, ±0.9%, ±0.8%, ±0.7%, ±0.6%, ±0.5%. In some embodiments, "approximately" and "about" refer to the listed amount, value, or duration ±1%. In some embodiments, "approximately" and "about" refer to the listed amount, value, or duration ±0.5%. In some embodiments, "approximately" and "about" refer to the listed amount, value, or duration ±0.1%.

### Detailed description

### Compounds of the disclosure

The present disclosure provides specific salt forms of phenyl pyrrole aminoguanidine compounds. By a "compound of the disclosure" is meant a compound as defined herein below.

AP1189 ((*E*)-*N*-[1-(2-nitrophenyl)-1-*H*-pyrrole-2-yl-allylideneamino]-guanidine) has the chemical structure of formula I, including tautomeric and stereoisomeric forms thereof:

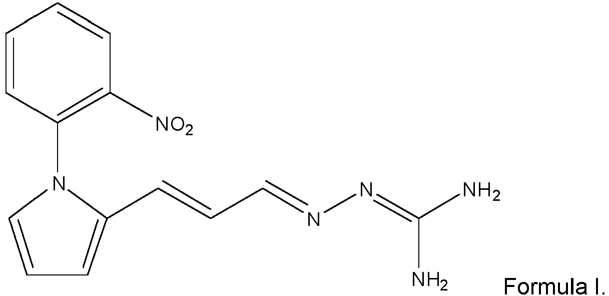

In some embodiments the compound is N"-[(E)-[(2E)-3-[1-(2-nitrophenyl)-1H-pyrrol-2-yl]prop-2-en-1-ylidene]amino]guanidinium acetate or N"-[(E)-[(2E)-3-[1-(2-nitrophenyl)-1H-pyrrol-2-yl]prop-2-en-1-ylidene]amino]guanidinium succinate as specified herein throughout.

In some embodiments of the present disclosure, the compound of the disclosure is a pharmaceutically acceptable salt of AP1189.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine) of formula I: 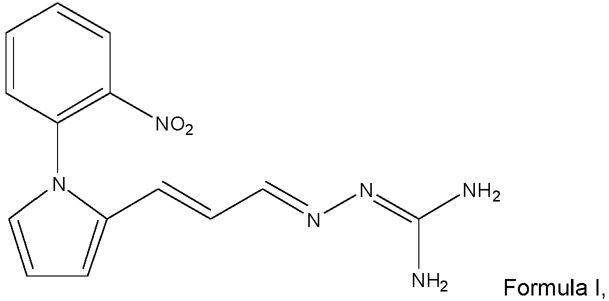 including tautomeric and stereoisomeric forms thereof, which is soluble at low pH, such as highly soluble at low pH.

In some embodiments of the present disclosure, there is provided a pharmaceutically acceptable salt of AP1189 which is soluble at low pH (e.g. acidic pH).

In some embodiments of the present disclosure, there is provided a pharmaceutically acceptable salt of AP1189 which is highly soluble at low pH (e.g. acidic pH), such as a pharmaceutically acceptable salt of AP1189 which has a high solubility at low pH.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, wherein said pharmaceutically acceptable salt has a high solubility at low pH.

In some embodiments said pharmaceutically acceptable salt of AP1189 has high solubility at low pH.

In some embodiments said pharmaceutically acceptable salt of AP1189 has high solubility at gastric pH.

Reference to 'high solubility' in some embodiments refer to 'highly soluble'.

Reference to gastric pH herein refers to the pH of the stomach (gastric juice, gastric acid), having a pH of approximately 1 to 3. Hydrochloric acid is secreted by gastric parietal cells to kill or reduce the growth of certain bacteria and to facilitate the denaturation of proteins as they enter the gastro-intestinal tract. Gastric acid is regulated in feedback systems to increase production when needed, such as after a meal. Other cells in the stomach produce bicarbonate, a base, to buffer the fluid, ensuring a regulated pH. These cells also produce mucus - a viscous barrier to prevent gastric acid from damaging the stomach. The pH of gastric acid is 1.5 to 3.5 in the human stomach lumen, a level maintained by the proton pump H⁺/K⁺ ATPase. The pH level rises when food enters the stomach (as high as pH 6), but it lowers again throughout digestion as stomach acid is secreted.

In some embodiments said pharmaceutically acceptable salt of AP1189 is soluble at a pH of 1 to 3, such as at a pH of 0.5 to 3.5. In some embodiments said pharmaceutically acceptable salt of AP1189 is soluble at a pH of 1.2.

In some embodiments said pharmaceutically acceptable salt of AP1189 has high solubility at a pH of 1 to 3, such as at a pH of 0.5 to 3.5.

In some embodiments said pharmaceutically acceptable salt of AP1189 has high solubility at a pH of 1.2.

In some embodiments said pharmaceutically acceptable salt of AP1189 has high solubility at pH 1.2.

In some embodiments said pharmaceutically acceptable salt of AP1189 has high solubility at acidic conditions. In some embodiments said pharmaceutically acceptable salt of AP1189 is highly soluble under acidic conditions.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which is highly soluble at low pH.

In some embodiments pharmaceutically acceptable salt of AP1189 are considered soluble or highly soluble at low pH when they have a solubility at or above a certain concentration (mM) at low pH, such as at acidic pH, such as a pH of 1.2.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of at least 10 mM at pH 1.2, such as at least 11 mM at pH 1.2, such as at least 12 mM at pH 1.2, such as at least 13 mM at pH 1.2, such as at least 14 mM at pH 1.2, such as at least 15 mM at pH 1.2, such as at least 16 mM at pH 1.2, such as at least 17 mM at pH 1.2, such as at least 18 mM at pH 1.2, such as at least 19 mM at pH 1.2, such as at least 20 mM at pH 1.2, such as at least 25 mM at pH 1.2, such as at least 30 mM at pH 1.2, such as at least 35 mM at pH 1.2, such as at least 40 mM at pH 1.2, such as at least 45 mM at pH 1.2, such as at least 50 mM at pH 1.2, such as at least 60 mM at pH 1.2, such as at least 70 mM at pH 1.2, such as at least 80 mM at pH 1.2, such as at least 90 mM at pH 1.2, such as at least 100 mM at pH 1.2, such as at least 110 mM at pH 1.2, such as at least 120 mM at pH 1.2, such as at least 130 mM at pH 1.2, such as at least 140 mM at pH 1.2, such as at least 150 mM at pH 1.2, such as at least 175 mM at pH 1.2, such as at least 200 mM at pH 1.2, such as at least 250 mM at pH 1.2, such as at least 300 mM at pH 1.2, such as at least 350 mM at pH 1.2, such as at least 400 mM at pH 1.2, such as at least 450 mM at pH 1.2, such as at least 500 mM at pH 1.2, such as at least 550 mM at pH 1.2.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of at least 10 mM at pH 1 to 3, such as at pH 0.5 to 3.5.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of at least 10 mM at pH 1.2.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of at least 15 mM at pH 1 to 3, such as at pH 0.5 to 3.5.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of at least 15 mM at pH 1.2.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of ≥ 15 mM to < 50 mM at pH 1 to 3, such as at pH 0.5 to 3.5.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of ≥ 15 mM to < 50 mM at pH 1.2.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of ≥ 50 mM at pH 1 to 3, such as at pH 0.5 to 3.5.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of ≥ 50 mM at pH 1.2.

In some embodiments of the present disclosure, there is provided a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, which has a solubility of at least 25 mg/mL at pH 1.2, such as at least 50 mg/mL at pH 1.2, such as at least 75 mg/mL at pH 1.2, such as at least 100 mg/mL at pH 1.2, such as at least 125 mg/mL at pH 1.2, such as at least 150 mg/mL at pH 1.2, such as at least 175 mg/mL at pH 1.2, such as at least 200 mg/mL at pH 1.2.

The present inventors have surprisingly found that a subset of pharmaceutically acceptable salts of AP1189 are highly soluble at low pH.

In particular, the present inventors have surprisingly found that the acetate salt of AP1189,the succinate salt of AP1189, the DL-mandelic acid salt of AP1189, the hippuric acid salt of AP1189, the L-lactic acid salt of AP1189, the besylate salt of AP1189, the oxoglutarate salt of AP1189, the formic acid salt of AP1189, the DL-lactic acid salt of AP1189, the glutaric acid salt of AP1189 and the adipic acid salt of AP1189 are highly soluble at low pH, such as highly soluble at pH 1.2. On the contrary, e.g. the tosylate salt of AP1189 is markedly less soluble at low pH.

In some embodiments of the present disclosure, the pharmaceutically acceptable salt of AP1189 has higher solubility at low pH such as a pH of 1.2 than AP1189 fumarate and/or AP1189 tosylate and/or AP1189 napadisylate, and/or AP1189 esylate, and/or AP1189 edisylate, and/or AP1189 cyclamate, and/or AP1189 oxalate, and/or AP1189 (+)-Camphor-10-sulfonic acid salt.

In some embodiments of the present disclosure, the pharmaceutically acceptable salt of AP1189 has higher solubility at low pH such as a pH of 1.2 than AP1189 tosylate.

### AP1189 soluble salts

In some embodiments, the pharmaceutically acceptable salt of AP1189 is selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof

In some embodiments of the present disclosure, the solubility of the AP1189 salt is at least 15 mM at pH low pH, such as at pH 1 to 3, such as at pH 0.5 to 3.5, such as at pH 1.2.

In some embodiments, the pharmaceutically acceptable salt of AP1189 is selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof.

In some embodiments of the present disclosure, the solubility of the AP1189 salt is at least 50 mM at pH low pH, such as at pH 1 to 3, such as at pH 0.5 to 3.5, such as at pH 1.2.

In some embodiments, the pharmaceutically acceptable salt of AP1189 according to the present disclosure is a crystalline or polymorphic form of a pharmaceutically acceptable salt of AP1189 selected from the group consisting of: AP1189 acetate and AP1189 succinate. Polymorphic forms are prepared and disclosed in WO 2022/268814.

### AP1189 acetate

In some embodiments, the pharmaceutically acceptable salt of AP1189 is (E)-N-[1-(2-nitrophenyl)-1-*H*-pyrrole-2-yl-allylideneamino]-guanidinium acetate, including tautomeric and stereoisomeric forms thereof (AP1189 acetate).

In some embodiments of the present disclosure, there is provided the acetate salt of AP1189 ((*E*)-*N*-[1-(2-nitrophenyl)-1-*H*-pyrrole-2-yl-allylideneamino]-guanidine), of formula II: 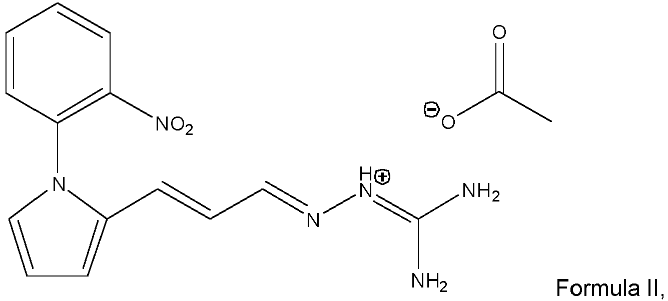 including tautomeric and stereoisomeric forms thereof.

In some embodiments said AP1189 acetate has high solubility at low pH, such as high solubility at a pH of 0.5 to 3.5, such as 1 to 3, such as high solubility at a pH of 1.2.

In some embodiments of the present disclosure, the solubility of the AP1189 acetate is at least 50 mM at pH 1.2, such as at least 60 mM at pH 1.2, such as at least 70 mM at pH 1.2, such as at least 80 mM at pH 1.2, such as at least 90 mM at pH 1.2, such as at least 100 mM at pH 1.2, such as at least 110 mM at pH 1.2, such as at least 120 mM at pH 1.2, such as at least 130 mM at pH 1.2, such as at least 140 mM at pH 1.2, such as at least 150 mM at pH 1.2, such as at least 175 mM at pH 1.2, such as at least 200 mM at pH 1.2, such as at least 250 mM at pH 1.2, such as at least 300 mM at pH 1.2, such as at least 350 mM at pH 1.2, such as at least 400 mM at pH 1.2, such as at least 450 mM at pH 1.2, such as at least 500 mM at pH 1.2, such as at least 550 mM at pH 1.2, such as at least 600 mM at pH 1.2.

In some embodiments of the present disclosure, the solubility of the AP1189 acetate is approx. 617 mM ± 200 mM at pH 1.2. In some embodiments of the present disclosure, the solubility of the AP1189 acetate is approx. 617 mM ± 100 mM at pH 1.2. In some embodiments of the present disclosure, the solubility of the AP1189 acetate is approx. 617 mM ± 50 mM at pH 1.2.

In some embodiments of the present disclosure, the solubility of the AP1189 acetate is at least 25 mg/mL at pH 1.2, such as at least 50 mg/mL at pH 1.2, such as at least 75 mg/mL at pH 1.2, such as at least 100 mg/mL at pH 1.2, such as at least 125 mg/mL at pH 1.2, such as at least 150 mg/mL at pH 1.2, such as at least 175 mg/mL at pH 1.2, such as at least 200 mg/mL at pH 1.2.

In some embodiments of the present disclosure, the solubility of the AP1189 acetate is approx. 223 mg/mL ± 100 mg/mL at pH 1.2. In some embodiments of the present disclosure, the solubility of the AP1189 acetate is approx. 223 mg/mL ± 75 mg/mL at pH 1.2. In some embodiments of the present disclosure, the solubility of the AP1189 acetate is approx. 223 mg/mL ± 50 mg/mL at pH 1.2. In some embodiments of the present disclosure, the solubility of the AP1189 acetate is approx. 223 mg/mL ± 25 mg/mL at pH 1.2.

### AP1189 succinate

In some embodiments, the pharmaceutically acceptable salt of AP1189 is (*E*)-*N*-[1-(2-nitrophenyl)-1-*H*-pyrrole-2-yl-allylideneamino]-guanidinium succinate, including tautomeric and stereoisomeric forms thereof (AP1189 succinate, SP1189).

In some embodiments of the present disclosure, there is provided the succinate salt of AP1189 ((*E*)-*N*-[1-(2-nitrophenyl)-1-*H*-pyrrole-2-yl-allylideneamino]-guanidine), of formula III: 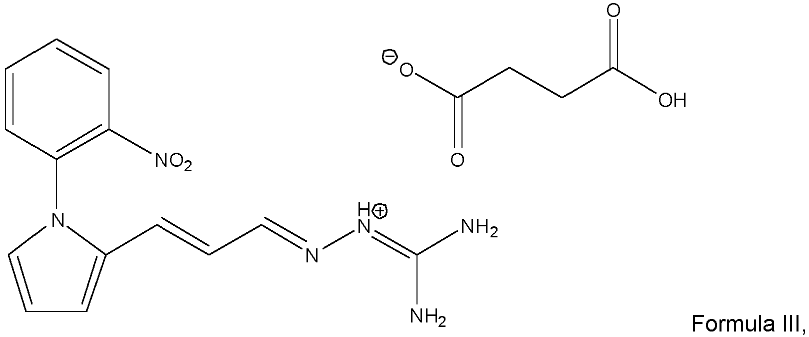 including tautomeric and stereoisomeric forms thereof.

In some embodiments said AP1189 succinate has high solubility at low pH, such as high solubility at a pH of 0.5 to 3.5, such as 1 to 3, such as high solubility at a pH of 1.2.

In some embodiments of the present disclosure, the solubility of the AP1189 succinate is at least 20 mM at pH 1.2, such as at least 30 mM at pH 1.2, such as at least 35 mM at pH 1.2, such as at least 40 mM at pH 1.2, such as at least 50 mM at pH 1.2, such as at least 60 mM at pH 1.2, such as at least 70 mM at pH 1.2, such as at least 80 mM at pH 1.2, such as at least 90 mM at pH 1.2, such as at least 100 mM at pH 1.2, such as at least 110 mM at pH 1.2, such as at least 120 mM at pH 1.2, such as at least 130 mM at pH 1.2, such as at least 140 mM at pH 1.2, such as at least 150 mM at pH 1.2, such as at least 175 mM at pH 1.2, such as at least 200 mM at pH 1.2, such as at least 250 mM at pH 1.2, such as at least 300 mM at pH 1.2, such as at least 350 mM at pH 1.2, such as at least 400 mM at pH 1.2, such as at least 450 mM at pH 1.2, such as at least 500 mM at pH 1.2, such as at least 550 mM at pH 1.2.

In some embodiments of the present disclosure, the solubility of the AP1189 succinate is approx. 593 mM ± 200 mM at pH 1.2. In some embodiments of the present disclosure, the solubility of the AP1189 succinate is approx. 593 mM ± 100 mM at pH 1.2. In some embodiments of the present disclosure, the solubility of the AP1189 succinate is approx. 593 mM ± 50 mM at pH 1.2.

In some embodiments of the present disclosure, the solubility of the AP1189 succinate is at least 25 mg/mL at pH 1.2, such as at least 50 mg/mL at pH 1.2, such as at least 75 mg/mL at pH 1.2, such as at least 100 mg/mL at pH 1.2, such as at least 125 mg/mL at pH 1.2, such as at least 150 mg/mL at pH 1.2, such as at least 175 mg/mL at pH 1.2, such as at least 200 mg/mL at pH 1.2.

In some embodiments of the present disclosure, the solubility of the AP1189 succinate is approx. 245 mg/mL ± 100 mg/mL at pH 1.2. In some embodiments of the present disclosure, the solubility of the AP1189 succinate is approx. 245 mg/mL ± 75 mg/mL at pH 1.2. In some embodiments of the present disclosure, the solubility of the AP1189 succinate is approx. 245 mg/mL ± 50 mg/mL at pH 1.2. In some embodiments of the present disclosure, the solubility of the AP1189 succinate is approx. 245 mg/mL ± 25 mg/mL at pH 1.2.

### Pharmaceutical compositions

It is also an aspect of the present disclosure to provide a pharmaceutical composition comprising a pharmaceutically acceptable salt of (E)-N-[1-(2-nitrophenyl)-1-H-pyrrole-2-yl-allylideneamino]-guanidine, including tautomeric and stereoisomeric forms thereof (AP1189).

In some embodiments the pharmaceutical composition comprises a compound selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof.

In some embodiments the pharmaceutical composition comprises a compound selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof.

In some embodiments the pharmaceutical composition comprises (*E*)-*N*-[1-(2-nitrophenyl)-1-*H*-pyrrole-2-yl-allylideneamino]-guanidinium acetate, including tautomeric and stereoisomeric forms thereof (AP1189 acetate).

In some embodiments the pharmaceutical composition comprises (*E*)-*N*-[1-(2-nitrophenyl)-1-*H*-pyrrole-2-yl-allylideneamino]-guanidinium succinate, including tautomeric and stereoisomeric forms thereof (AP1189 succinate, 'SP1189').

The terms "composition" and "pharmaceutical composition" may be used interchangeably herein.

It is also an aspect of the present disclosure to provide a unit dosage form of a pharmaceutical composition comprising a pharmaceutically acceptable salt of (E)-N-[1-(2-nitrophenyl)-1-H-pyrrole-2-yl-allylideneamino]-guanidine (AP1189).

In some embodiments the unit dosage form comprises a pharmaceutical composition comprising a pharmaceutically acceptable salt of AP1189 selected from the group consisting of the acetate salt of AP1189, and the succinate salt of AP1189.

In some embodiments the unit dosage form comprises a pharmaceutical composition comprising (*E*)-*N*-[1-(2-nitrophenyl)-1-*H*-pyrrole-2-yl-allylideneamino]-guanidinium acetate, including tautomeric and stereoisomeric forms thereof (AP1189 acetate).

In some embodiments the unit dosage form comprises a pharmaceutical composition comprising (*E*)-*N*-[1-(2-nitrophenyl)-1-*H*-pyrrole-2-yl-allylideneamino]-guanidinium succinate, including tautomeric and stereoisomeric forms thereof (AP1189 succinate, SP1189).

### Oral formulation

It is an aspect of the present disclosure to provide an oral formulation comprising a soluble form of AP1189, or a pharmaceutically acceptable salt thereof, which is soluble at low pH, such as has high solubility at low pH.

It is an aspect of the present disclosure to provide an oral formulation comprising a pharmaceutically acceptable salt of AP1189 ((*E*)-N-[1-(2-nitrophenyl)-1-*H*-pyrrole-2-yl-allylideneamino]-guanidine) of formula I: 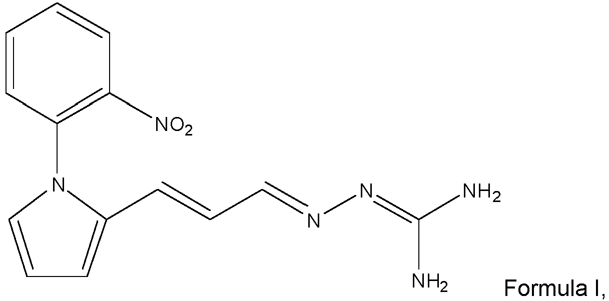 including tautomeric and stereoisomeric forms thereof, wherein said pharmaceutically acceptable salt is soluble at low pH, such as has high solubility at low pH.

Also disclosed is an oral formulation comprising a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, wherein said pharmaceutically acceptable salt is soluble at low pH, such as has high solubility at low pH, and at least one pharmaceutically acceptable excipient.

In some embodiments said oral formulation comprises a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, wherein said pharmaceutically acceptable salt has a solubility of at least 10 mM at pH 1.2, such as at least 11 mM at pH 1.2, such as at least 12 mM at pH 1.2, such as at least 13 mM at pH 1.2, such as at least 14 mM at pH 1.2, such as at least 15 mM at pH 1.2, such as at least 16 mM at pH 1.2, such as at least 17 mM at pH 1.2, such as at least 18 mM at pH 1.2, such as at least 19 mM at pH 1.2, such as at least 20 mM at pH 1.2, such as at least 25 mM at pH 1.2, such as at least 30 mM at pH 1.2, such as at least 35 mM at pH 1.2, such as at least 40 mM at pH 1.2, such as at least 50 mM at pH 1.2, such as at least 60 mM at pH 1.2, such as at least 70 mM at pH 1.2, such as at least 80 mM at pH 1.2, such as at least 90 mM at pH 1.2, such as at least 100 mM at pH 1.2, such as at least 110 mM at pH 1.2, such as at least 120 mM at pH 1.2, such as at least 130 mM at pH 1.2, such as at least 140 mM at pH 1.2, such as at least 150 mM at pH 1.2, such as at least 175 mM at pH 1.2, such as at least 200 mM at pH 1.2, such as at least 250 mM at pH 1.2, such as at least 300 mM at pH 1.2, such as at least 350 mM at pH 1.2, such as at least 400 mM at pH 1.2, such as at least 450 mM at pH 1.2, such as at least 500 mM at pH 1.2, such as at least 550 mM at pH 1.2.

In some embodiments said oral formulation comprises a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, wherein said pharmaceutically acceptable salt has a solubility of at least 10 mM at pH 1.2; such as at least 15 mM at pH 1.2, such as a solubility of ≥ 15 to < 50 mM at pH 1.2, such as a solubility of ≥ 50 mM.

In some embodiments said oral formulation comprises a pharmaceutically acceptable salt of AP1189 ((*E*)-*N*-[1-(2-nitrophenyl)-1-*H*-pyrrole-2-yl-allylideneamino]-guanidine), including tautomeric and stereoisomeric forms thereof, wherein said pharmaceutically acceptable salt has a solubility of at least 25 mg/mL at pH 1.2, such as at least 50 mg/mL at pH 1.2, such as at least 75 mg/mL at pH 1.2, such as at least 100 mg/mL at pH 1.2, such as at least 125 mg/mL at pH 1.2, such as at least 150 mg/mL at pH 1.2, such as at least 175 mg/mL at pH 1.2, such as at least 200 mg/mL at pH 1.2.

It is also an aspect of the present disclosure to provide an oral formulation comprising a pharmaceutically acceptable salt of AP1189 selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and at least one pharmaceutically acceptable excipient.

It is also an aspect of the present disclosure to provide an oral formulation comprising a pharmaceutically acceptable salt of AP1189 selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and at least one pharmaceutically acceptable excipient.

It is also an aspect of the present disclosure to provide an oral formulation comprising (*E*)-*N*-[1-(2-nitrophenyl)-1-*H*-pyrrole-2-yl-allylideneamino]-guanidinium acetate, including tautomeric and stereoisomeric forms thereof (AP1189 acetate), and at least one pharmaceutically acceptable excipient.

It is also an aspect of the present disclosure to provide an oral formulation comprising (*E*)-*N*-[1-(2-nitrophenyl)-1-*H*-pyrrole-2-yl-allylideneamino]-guanidinium succinate, including tautomeric and stereoisomeric forms thereof (AP1189 succinate, SP1189), and at least one pharmaceutically acceptable excipient.

In some embodiments said oral formulation delivers said pharmaceutically acceptable salt of AP1189 to the gastric compartment (or stomach). In some embodiments said oral formulation primarily or predominantly delivers said pharmaceutically acceptable salt of AP1189 to the gastric compartment (or stomach).

In some embodiments said oral formulation releases said pharmaceutically acceptable salt of AP1189 in the gastric compartment. In some embodiments said oral formulation primarily or predominantly releases said pharmaceutically acceptable salt of AP1189 in the gastric compartment.

In some embodiments said oral formulation releases said pharmaceutically acceptable salt of AP1189 in the gastric compartment by immediate release, by delayed release, by burst release, or by any means of releasing said pharmaceutically acceptable salt of AP1189 primarily or predominantly in the gastric compartment.

In some embodiments said oral formulation delivers and/or releases not less than 65% to 80% of the pharmaceutically acceptable salt of AP1189 in the gastric compartment.

In some embodiments said oral formulation delivers and/or releases not less than 65% of the pharmaceutically acceptable salt of AP1189, such as not less than 70%, such as not less than 75%, such as not less than 80%, such as not less than 85%, such as not less than 90%, such as not less than 95% of the pharmaceutically acceptable salt of AP1189 in the gastric compartment.

In some embodiments said oral formulation immediately releases said pharmaceutically acceptable salt of AP1189 in the gastric compartment.

In some embodiments said oral formulation releases said pharmaceutically acceptable salt of AP1189 in the gastric compartment for gastric absorption of said AP1189.

In some embodiments said oral formulation releases said pharmaceutically acceptable salt of AP1189 in the gastric compartment for absorption of said AP1189 over the gastric mucus layer.

In some embodiments said oral formulation is designed for gastric delivery.

In some embodiments said oral formulation is designed for gastric release.

In some embodiments said oral formulation is designed for gastric absorption.

In some embodiments said oral formulation is non-alkaline. In some embodiments said oral formulation is not a suspension. In some embodiments said oral formulation is not an alkaline suspension. In some embodiments said oral formulation is not an enteric coated tablet.

In some embodiments said oral formulation is a solid oral formulation.

In a preferred embodiment said oral formulation is a solid oral formulation.

It is an aspect of the present disclosure to provide a solid oral formulation comprising a pharmaceutically acceptable salt of (*E*)-*N*-[1-(2-nitrophenyl)-1-*H*-pyrrole-2-yl-allylideneamino]-guanidinium, including tautomeric and stereoisomeric forms thereof, and at least one pharmaceutically acceptable excipient, wherein said pharmaceutically acceptable salt is soluble or highly soluble at low pH,
such as wherein the pharmaceutically acceptable salt of AP1189 has a solubility of at least 10 mM at pH 1.2, such as at least 11 mM at pH 1.2, such as at least 12 mM at pH 1.2, such as at least 13 mM at pH 1.2, such as at least 14 mM at pH 1.2, such as at least 15 mM at pH 1.2, such as at least 16 mM at pH 1.2, such as at least 17 mM at pH 1.2, such as at least 18 mM at pH 1.2, such as at least 19 mM at pH 1.2, such as at least 20 mM at pH 1.2, such as at least 25 mM at pH 1.2, such as at least 30 mM at pH 1.2, such as at least 35 mM at pH 1.2, such as at least 40 mM at pH 1.2, such as at least 50 mM at pH 1.2, such as at least 60 mM at pH 1.2, such as at least 70 mM at pH 1.2, such as at least 80 mM at pH 1.2, such as at least 90 mM at pH 1.2, such as at least 100 mM at pH 1.2, such as at least 110 mM at pH 1.2, such as at least 120 mM at pH 1.2, such as at least 130 mM at pH 1.2, such as at least 140 mM at pH 1.2, such as at least 150 mM at pH 1.2, such as at least 175 mM at pH 1.2, such as at least 200 mM at pH 1.2, such as at least 250 mM at pH 1.2, such as at least 300 mM at pH 1.2, such as at least 350 mM at pH 1.2, such as at least 400 mM at pH 1.2, such as at least 450 mM at pH 1.2, such as at least 500 mM at pH 1.2, such as at least 550 mM at pH 1.2; or such as wherein the pharmaceutically acceptable salt of AP1189 has a solubility of at least 25 mg/mL at pH 1.2, such as at least 50 mg/mL at pH 1.2, such as at least 75 mg/mL at pH 1.2, such as at least 100 mg/mL at pH 1.2, such as at least 125 mg/mL at pH 1.2, such as at least 150 mg/mL at pH 1.2, such as at least 175 mg/mL at pH 1.2, such as at least 200 mg/mL at pH 1.2.

It is an aspect of the present disclosure to provide a solid oral formulation comprising a pharmaceutically acceptable salt of (*E*)-*N*-[1-(2-nitrophenyl)-1-*H*-pyrrole-2-yl-allylideneamino]-guanidinium, including tautomeric and stereoisomeric forms thereof, and at least one pharmaceutically acceptable excipient, wherein said pharmaceutically acceptable salt has a solubility of at least 10 mM at pH 1.2; such as at least 15 mM at pH 1.2, such as a solubility of ≥ 15 to < 50 mM at pH 1.2, such as a solubility of ≥ 50 mM.

In some embodiments of the present disclosure there is provided a solid oral formulation comprising a pharmaceutically acceptable salt of AP1189 selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and at least one pharmaceutically acceptable excipient.

It is an aspect of the present disclosure to provide a solid oral formulation comprising (*E*)-*N*-[1-(2-nitrophenyl)-1-*H*-pyrrole-2-yl-allylideneamino]-guanidinium acetate, including tautomeric and stereoisomeric forms thereof (AP1189 acetate), and at least one pharmaceutically acceptable excipient.

It is an aspect of the present disclosure to provide a solid oral formulation comprising (*E*)-*N*-[1-(2-nitrophenyl)-1-*H*-pyrrole-2-yl-allylideneamino]-guanidinium succinate, including tautomeric and stereoisomeric forms thereof (AP1189 succinate, SP1189), and at least one pharmaceutically acceptable excipient.

In some embodiments said solid oral formulation is a solid oral dosage form.

In some embodiments said solid oral formulation is a delayed release solid oral formulation comprising a pharmaceutically acceptable salt of AP1189 targeting release in the gastric compartment.

In some embodiments said solid oral formulation is an immediate release solid oral formulation.

In some embodiments said solid oral dosage form is an immediate release solid oral dosage form.

In some embodiments said oral formulation is a tablet. In some embodiments said solid oral formulation is a tablet. In some embodiments said solid oral dosage form is a tablet.

It is an aspect of the present disclosure to provide a solid oral dosage form, such as a tablet, comprising a pharmaceutically acceptable salt of AP1189 selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and at least one pharmaceutically acceptable excipient.

In a preferred embodiment the present disclosure provides a solid oral dosage form, such as a tablet, comprising a pharmaceutically acceptable salt of AP1189 selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and at least one pharmaceutically acceptable excipient.

It is an aspect of the present disclosure to provide a solid oral dosage form, such as a tablet, comprising (*E*)-*N*-[1-(2-nitrophenyl)-1-*H*-pyrrole-2-yl-allylideneamino]-guanidinium acetate, including tautomeric and stereoisomeric forms thereof (AP1189 acetate), and at least one pharmaceutically acceptable excipient.

It is an aspect of the present disclosure to provide a solid oral dosage form, such as a tablet, comprising (*E*)-*N*-[1-(2-nitrophenyl)-1-*H*-pyrrole-2-yl-allylideneamino]-guanidinium succinate, including tautomeric and stereoisomeric forms thereof (AP1189 succinate, SP1189), and at least one pharmaceutically acceptable excipient.

In some embodiments there is provided a solid oral formulation, solid oral dosage form or tablet, comprising a pharmaceutically acceptable salt of AP1189 as disclosed herein, wherein not less than 65% to 80% of the pharmaceutically acceptable salt of AP1189 is dissolved into solution in the gastric compartment.

In some embodiments there is provided a solid oral formulation, solid oral dosage form or tablet, comprising a pharmaceutically acceptable salt of AP1189 as disclosed herein, wherein not less than 65%, such as not less than 70%, such as not less than 75%, such as not less than 80%, such as not less than 85%, such as not less than 90%, such as not less than 95% of the pharmaceutically acceptable salt of AP1189 is dissolved into solution in the gastric compartment.

In some embodiments said oral formulation is a delayed release tablet comprising a pharmaceutically acceptable salt of AP1189 as disclosed herein targeting release in the gastric compartment.

In some embodiments said oral formulation is a gastric retentive delayed-release formulation. In some embodiments said oral formulation is a gastroretentive tablet. In some embodiments said gastroretentive tablet is a gastroretentive double-layered tablet formulation. In some embodiments said gastroretentive double-layered tablet formulation is a gastric swelling system (GSS), such as a GSS consisting of a swelling layer and a drug release layer.

It is an aspect of the present disclosure to provide a gastric retentive delayed release solid oral formulation, dosage form or tablet, comprising a pharmaceutically acceptable salt of AP1189 selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and at least one pharmaceutically acceptable excipient.

It is an aspect of the present disclosure to provide a gastric retentive delayed release solid oral formulation, dosage form or tablet, comprising (*E*)-*N*-[1-(2-nitrophenyl)-1-*H-*pyrrole-2-yl-allylideneamino]-guanidinium acetate, including tautomeric and stereoisomeric forms thereof (AP1189 acetate), and at least one pharmaceutically acceptable excipient.

It is an aspect of the present disclosure to provide a gastric retentive delayed release solid oral formulation, dosage form or tablet, comprising (*E*)-*N*-[1-(2-nitrophenyl)-1-*H-*pyrrole-2-yl-allylideneamino]-guanidinium succinate, including tautomeric and stereoisomeric forms thereof (AP1189 succinate, SP1189), and at least one pharmaceutically acceptable excipient.

In some embodiments said oral formulation is an immediate release tablet. In some embodiments said solid oral formulation is an immediate release tablet.

It is an aspect of the present disclosure to provide an immediate release solid oral formulation, such as an immediate release solid oral dosage form, such as an immediate release tablet, comprising a pharmaceutically acceptable salt of AP1189 selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and at least one pharmaceutically acceptable excipient.

It is an aspect of the present disclosure to provide an immediate release solid oral formulation, such as an immediate release solid oral dosage form, such as an immediate release tablet, comprising a pharmaceutically acceptable salt of AP1189 selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and at least one pharmaceutically acceptable excipient.

It is an aspect of the present disclosure to provide an immediate release solid oral formulation, such as an immediate release solid oral dosage form, such as an immediate release tablet, comprising (*E*)-*N*-[1-(2-nitrophenyl)-1-*H*-pyrrole-2-yl-allylideneamino]-guanidinium acetate, including tautomeric and stereoisomeric forms thereof (AP1189 acetate), and at least one pharmaceutically acceptable excipient.

It is an aspect of the present disclosure to provide an immediate release solid oral formulation, such as an immediate release solid oral dosage form, such as an immediate release tablet, comprising (*E*)-*N*-[1-(2-nitrophenyl)-1-*H*-pyrrole-2-yl-allylideneamino]-guanidinium succinate, including tautomeric and stereoisomeric forms thereof (AP1189 succinate, SP1189), and at least one pharmaceutically acceptable excipient.

In some embodiments there is provided an oral formulation, such as a solid oral formulation, such as an immediate-release solid oral formulation, comprising a pharmaceutically acceptable salt of AP1189 as disclosed herein, wherein not less than 65% to 80% of the pharmaceutically acceptable salt of AP1189 is dissolved into solution in 5 minutes.

In some embodiments there is provided an oral formulation, such as a solid oral formulation, such as an immediate-release solid oral formulation, comprising AP1189 acetate, or AP1189 succinate, wherein not less than 65% to 80% of the AP1189 acetate or the AP1189 succinate is dissolved into solution in 5 minutes.

In some embodiments there is provided an oral formulation, such as a solid oral formulation, such as an immediate-release solid oral formulation, comprising AP1189 acetate, or AP1189 succinate, wherein not less than 65% to 80% of the AP1189 acetate or the AP1189 succinate is dissolved into solution in 5 minutes.

In some embodiments there is provided an oral formulation, such as a solid oral formulation, such as an immediate-release solid oral formulation, comprising AP1189 acetate or AP1189 succinate, wherein not less than 65% to 80% of the AP1189 acetate or the AP1189 succinate is dissolved into solution in 5 minutes.

In some embodiments there is provided an oral formulation, such as a solid oral formulation, such as an immediate-release solid oral formulation, comprising a pharmaceutically acceptable salt of AP1189 as disclosed herein, wherein not less than 65 to 80% of the pharmaceutically acceptable salt of AP1189 is dissolved into solution in 5 minutes at a pH of 1 to 3, such as a pH of 0.5 to 3.5; such as a pH of 0.5 to 1, such as a pH of 1 to 1.5, such as a pH of 1.5 to 2, such as a pH of 2 to 2.5, such as a pH of 2.5 to 3, such as a pH of 3 to 3.5.

In some embodiments there is provided an oral formulation, such as a solid oral formulation, such as an immediate-release solid oral formulation, comprising a pharmaceutically acceptable salt of AP1189 selected from the group consisting of AP1189 acetate, and AP1189 succinate, wherein not less than 65 to 80% of the pharmaceutically acceptable salt of AP1189 is dissolved into solution in 5 minutes at a pH of 1 to 3, such as a pH of 0.5 to 3.5; such as a pH of 0.5 to 1, such as a pH of 1 to 1.5, such as a pH of 1.5 to 2, such as a pH of 2 to 2.5, such as a pH of 2.5 to 3, such as a pH of 3 to 3.5.

In some embodiments there is provided an oral formulation, such as a solid oral formulation, such as an immediate-release solid oral formulation, comprising AP1189 acetate or AP1189 succinate, wherein not less than 65 to 80% of the AP1189 acetate or the AP1189 succinate is dissolved into solution in 5 minutes at a pH of 1 to 3, such as a pH of 0.5 to 3.5; such as a pH of 0.5 to 1, such as a pH of 1 to 1.5, such as a pH of 1.5 to 2, such as a pH of 2 to 2.5, such as a pH of 2.5 to 3, such as a pH of 3 to 3.5.

In some embodiments there is provided an oral formulation, such as a solid oral formulation, such as an immediate-release solid oral formulation, comprising a pharmaceutically acceptable salt of AP1189 as disclosed herein, wherein not less than 65 to 80% of the pharmaceutically acceptable salt of AP1189 is dissolved into solution in 5 minutes at a pH of 1.2.

In some embodiments there is provided an oral formulation, such as a solid oral formulation, such as an immediate-release solid oral formulation, comprising a pharmaceutically acceptable salt of AP1189 selected from the group consisting of AP1189 acetate, and AP1189 succinate, wherein not less than 65 to 80% of the pharmaceutically acceptable salt of AP1189 is dissolved into solution in 5 minutes at a pH of 1.2.

In some embodiments there is provided an oral formulation, such as a solid oral formulation, such as an immediate-release solid oral formulation, comprising AP1189 acetate or AP1189 succinate, wherein not less than 65 to 80% of the AP1189 acetate or the AP1189 succinate is dissolved into solution in 5 minutes at a pH of 1.2.

In some embodiments there is provided an oral formulation, such as a solid oral formulation, such as an immediate-release solid oral formulation, comprising a pharmaceutically acceptable salt of AP1189 as disclosed herein, wherein not less than 65% to 80% of the of the nominal dose of AP1189 is released in 5 minutes in a monograph dissolution test.

In some embodiments there is provided an oral formulation, such as a solid oral formulation, such as an immediate-release solid oral formulation, comprising a pharmaceutically acceptable salt of AP1189 selected from the group consisting of AP1189 acetate, and AP1189 succinate, wherein not less than 65% to 80% of the of the nominal dose of AP1189 is released in 5 minutes in a monograph dissolution test.

In some embodiments there is provided an oral formulation, such as a solid oral formulation, such as an immediate-release solid oral formulation, comprising AP1189 acetate or AP1189 succinate, wherein not less than 65% to 80% of the of the nominal dose of AP1189 is released in 5 minutes in a monograph dissolution test.

In some embodiments there is provided an oral formulation, such as a solid oral formulation, such as an immediate-release solid oral formulation, comprising a pharmaceutically acceptable salt of AP1189 as disclosed herein, wherein not less than 65% to 80% of the pharmaceutically acceptable salt of AP1189 is dissolved into solution in 5 minutes at pH 1 (0.1 N HCl).

In some embodiments there is provided an oral formulation, such as a solid oral formulation, such as an immediate-release solid oral formulation, comprising a pharmaceutically acceptable salt of AP1189 as disclosed herein, wherein not less than 65% to 80% of the pharmaceutically acceptable salt of AP1189 is dissolved into solution in 5 minutes at pH 1.

In some embodiments there is provided an oral formulation, such as a solid oral formulation, such as an immediate-release solid oral formulation, comprising a pharmaceutically acceptable salt of AP1189 selected from the group consisting of AP1189 acetate and AP1189 succinate, wherein not less than 65 to 80% of the pharmaceutically acceptable salt of AP1189 is dissolved into solution in 5 minutes at pH 1.

In some embodiments there is provided an oral formulation, such as a solid oral formulation, such as an immediate-release solid oral formulation, comprising AP1189 acetate or AP1189 succinate, wherein not less than 65 to 80% of the AP1189 acetate or the AP1189 succinate is dissolved into solution in 5 minutes at pH 1.

In some embodiments there is provided an oral formulation, such as a solid oral formulation, such as an immediate-release solid oral formulation, comprising a pharmaceutically acceptable salt of AP1189 as disclosed herein, wherein not less than 80% of the pharmaceutically acceptable salt of AP1189 is dissolved into solution in 10 minutes.

In some embodiments there is provided an oral formulation, such as a solid oral formulation, such as an immediate-release solid oral formulation, comprising a pharmaceutically acceptable salt of AP1189 as disclosed herein, wherein not less than 80% of the pharmaceutically acceptable salt of AP1189 is dissolved into solution in 15 minutes.

In some embodiments there is provided an oral formulation, such as a solid oral formulation, such as an immediate-release solid oral formulation, comprising a pharmaceutically acceptable salt of AP1189 selected from the group consisting of AP1189 acetate and AP1189 succinate, wherein not less than 80% of the pharmaceutically acceptable salt of AP1189 is dissolved into solution in 10 minutes, such as 15 minutes.

In some embodiments there is provided an oral formulation, such as a solid oral formulation, such as an immediate-release solid oral formulation, comprising AP1189 acetate or AP1189 succinate, wherein not less than 80% of the AP1189 acetate or the AP1189 succinate is dissolved into solution in 10 minutes.

In some embodiments there is provided an oral formulation, such as a solid oral formulation, such as an immediate-release solid oral formulation, comprising AP1189 acetate or AP1189 succinate, wherein not less than 80% of the AP1189 acetate or the AP1189 succinate is dissolved into solution in 15 minutes.

In some embodiments there is provided an oral formulation, such as a solid oral formulation, such as an immediate-release solid oral formulation, comprising a pharmaceutically acceptable salt of AP1189 as disclosed herein, wherein not less than 80% of the pharmaceutically acceptable salt of AP1189 is dissolved into solution in 10 minutes at a pH of 1 to 3, such as a pH of 0.5 to 3.5; such as a pH of 0.5 to 1, such as a pH of 1 to 1.5, such as a pH of 1.5 to 2, such as a pH of 2 to 2.5, such as a pH of 2.5 to 3, such as a pH of 3 to 3.5,
such as dissolved into solution in 15 minutes at a pH of 1 to 3, such as a pH of 0.5 to 3.5; such as a pH of 0.5 to 1, such as a pH of 1 to 1.5, such as a pH of 1.5 to 2, such as a pH of 2 to 2.5, such as a pH of 2.5 to 3, such as a pH of 3 to 3.5.

In some embodiments there is provided an oral formulation, such as a solid oral formulation, such as an immediate-release solid oral formulation, comprising a pharmaceutically acceptable salt of AP1189 selected from the group consisting of AP1189 acetate and AP1189 succinate, wherein not less than 80% of the pharmaceutically acceptable salt of AP1189 is dissolved into solution in 10 minutes at a pH of 1 to 3, such as a pH of 0.5 to 3.5; such as a pH of 0.5 to 1, such as a pH of 1 to 1.5, such as a pH of 1.5 to 2, such as a pH of 2 to 2.5, such as a pH of 2.5 to 3, such as a pH of 3 to 3.5,
such as dissolved into solution in 15 minutes at a pH of 1 to 3, such as a pH of 0.5 to 3.5; such as a pH of 0.5 to 1, such as a pH of 1 to 1.5, such as a pH of 1.5 to 2, such as a pH of 2 to 2.5, such as a pH of 2.5 to 3, such as a pH of 3 to 3.5.

In some embodiments there is provided an oral formulation, such as a solid oral formulation, such as an immediate-release solid oral formulation, comprising AP1189 acetate or AP1189 succinate, wherein not less than 80% of the AP1189 acetate or the AP1189 succinate is dissolved into solution in 10 minutes at a pH of 1 to 3, such as a pH of 0.5 to 3.5; such as a pH of 0.5 to 1, such as a pH of 1 to 1.5, such as a pH of 1.5 to 2, such as a pH of 2 to 2.5, such as a pH of 2.5 to 3, such as a pH of 3 to 3.5.

In some embodiments there is provided an oral formulation, such as a solid oral formulation, such as an immediate-release solid oral formulation, comprising a pharmaceutically acceptable salt of AP1189 as disclosed herein, wherein not less than 80% of the pharmaceutically acceptable salt of AP1189 is dissolved into solution in 10 minutes at a pH of 1.2, such as in 15 minutes at a pH of 1.2.

In some embodiments there is provided an oral formulation, such as a solid oral formulation, such as an immediate-release solid oral formulation, comprising a pharmaceutically acceptable salt of AP1189 selected from the group consisting of AP1189 acetate and AP1189 succinate, wherein not less than 80% of the pharmaceutically acceptable salt of AP1189 is dissolved into solution in 10 minutes at a pH of 1.2, such as in 15 minutes at a pH of 1.2.

In some embodiments there is provided an oral formulation, such as a solid oral formulation, such as an immediate-release solid oral formulation, comprising AP1189 acetate or AP1189 succinate, wherein not less than 80% of the AP1189 acetate or the AP1189 succinate is dissolved into solution in 10 minutes at a pH of 1.2.

In some embodiments there is provided an oral formulation, such as a solid oral formulation, such as an immediate-release solid oral formulation, comprising a pharmaceutically acceptable salt of AP1189 as disclosed herein, wherein not less than 80% of the of the nominal dose of AP1189 is released in 10 minutes in a monograph dissolution test.

In some embodiments there is provided an oral formulation, such as a solid oral formulation, such as an immediate-release solid oral formulation, comprising a pharmaceutically acceptable salt of AP1189 selected from the group consisting of AP1189 acetate and AP1189 succinate, wherein not less than 80% of the of the nominal dose of AP1189 is released in 10 minutes in a monograph dissolution test.

In some embodiments there is provided an oral formulation, such as a solid oral formulation, such as an immediate-release solid oral formulation, comprising AP1189 acetate or AP1189 succinate, wherein not less than 80% of the of the nominal dose of AP1189 is released in 10 minutes in a monograph dissolution test.

In some embodiments there is provided an oral formulation, such as a solid oral formulation, such as an immediate-release solid oral formulation, comprising a pharmaceutically acceptable salt of AP1189 as disclosed herein, wherein not less than 80% of the pharmaceutically acceptable salt of AP1189 is dissolved into solution in 10 minutes at a pH of 1, such as in 15 minutes at a pH of 1.

In some embodiments there is provided an oral formulation, such as a solid oral formulation, such as an immediate-release solid oral formulation, comprising a pharmaceutically acceptable salt of AP1189 selected from the group consisting of AP1189 acetate and AP1189 succinate, wherein not less than 80% of the pharmaceutically acceptable salt of AP1189 is dissolved into solution in 10 minutes at a pH of 1, such as in 15 minutes at a pH of 1.

In some embodiments there is provided an oral formulation, such as a solid oral formulation, such as an immediate-release solid oral formulation, comprising AP1189 acetate or AP1189 succinate, wherein not less than 80% of the AP1189 acetate or the AP1189 succinate is dissolved into solution in 10 minutes at a pH of 1.

In some embodiments there is provided an oral formulation, such as a solid oral formulation, such as an immediate-release solid oral formulation, comprising a pharmaceutically acceptable salt of AP1189 as disclosed herein, wherein the disintegration time of said oral formulation such as solid oral formulation is from ½ minute to 10 minutes, such as ½ minute to 1 minute, such as 1 to 2 minutes, such as 2 to 3 minutes, such as 3 to 4 minutes, such as 4 to 5 minutes, such as 5 to 6 minutes, such as 6 to 7 minutes, such as 7 to 8 minutes, such as 8 to 9 minutes, such as 9 to 10 minutes.

In some embodiments there is provided an oral formulation, such as a solid oral formulation, such as an immediate-release solid oral formulation, comprising a pharmaceutically acceptable salt of AP1189 selected from the group consisting of AP1189 acetate and AP1189 succinate, wherein the disintegration time of said oral formulation such as solid oral formulation is from ½ minute to 10 minutes, such as ½ minute to 1 minute, such as 1 to 2 minutes, such as 2 to 3 minutes, such as 3 to 4 minutes, such as 4 to 5 minutes, such as 5 to 6 minutes, such as 6 to 7 minutes, such as 7 to 8 minutes, such as 8 to 9 minutes, such as 9 to 10 minutes.

In some embodiments there is provided an oral formulation, such as a solid oral formulation, such as an immediate-release solid oral formulation, comprising AP1189 acetate or AP1189 succinate, wherein the disintegration time of said oral formulation such as solid oral formulation is from ½ minute to 10 minutes, such as ½ minute to 1 minute, such as 1 to 2 minutes, such as 2 to 3 minutes, such as 3 to 4 minutes, such as 4 to 5 minutes, such as 5 to 6 minutes, such as 6 to 7 minutes, such as 7 to 8 minutes, such as 8 to 9 minutes, such as 9 to 10 minutes.

In some embodiments said oral formulation such as solid oral formulation provides for a Tₘₐₓ of 1 to 6 hours post-dosing, such as 1 to 4 hours post dosing, such as 1 to 3 hours post dosing, such as 1 to 2 hours post dosing.

In some embodiments said oral formulation such as solid oral formulation provides for a Tₘₐₓ of 6 hours post-dosing, such as 5 hours post dosing, such as 4 hours post dosing, such as 3 hours post dosing, such as 2.5 hours post dosing, such as 2 hours post dosing, such as 1 hour post dosing.

In some embodiments there is provided an oral formulation, such as a solid oral formulation, comprising a pharmaceutically acceptable salt of AP1189 selected from the group consisting of AP1189 acetate and AP1189 succinate, wherein Tₘₐₓ is 1 to 4 hours post dosing, such as 1 to 3 hours post dosing, such as 1 to 2 hours post dosing.

In some embodiments there is provided an oral formulation, such as a solid oral formulation, comprising AP1189 acetate or AP1189 succinate, wherein Tₘₐₓ is 1 to 4 hours post dosing, such as 1 to 3 hours post dosing, such as 1 to 2 hours post dosing.

In some embodiments the elimination half-life of said said oral formulation, such as solid oral formulation, is 5 to 25 hours, such as 5 to 10 hours, such as 10 to 15 hours, such as 15 to 20 hours, such as 20 to 25 hours.

In some embodiments the elimination half-life of said said oral formulation, such as solid oral formulation, is 4 to 5 hours.

In some embodiments the elimination half-life of said said oral formulation, such as solid oral formulation, is 15 hours, such as 16 hours, such as 17 hours, such as 18 hours, such as 19 hours, such as 20 hours, such as 21 hours, such as 22 hours, such as 23 hours, such as 24 hours, such as 25 hours.

In some embodiments the exposure (Cₘₐₓ and/or AUCs) is higher for the solid oral formulation comprising AP1189, or a pharmaceutically acceptable salt thereof, than the exposure for an enteric coated tablet and/or for an alkaline suspension comprising AP1189, or a pharmaceutically acceptable salt thereof.

In some embodiments the exposure (Cₘₐₓ and/or AUCs) for the solid oral formulation comprising AP1189, or a pharmaceutically acceptable salt thereof, is comparable to the exposure for an alkaline suspension comprising AP1189, or a pharmaceutically acceptable salt thereof.

In some embodiments the exposure (Cₘₐₓ and/or AUCs) is higher for the solid oral formulation comprising AP1189 acetate or AP1189 succinate, than the exposure for an enteric coated tablet and/or for an alkaline suspension comprising AP1189 acetate or AP1189 succinate.

In some embodiments the exposure (Cₘₐₓ and/or AUCs) for the solid oral formulation comprising AP1189 acetate or AP1189 succinate, is comparable to the exposure for an alkaline suspension comprising AP1189 acetate or AP1189 succinate.

In some embodiments the relative bioavailability for the solid oral formulation comprising a pharmaceutically acceptable salt of AP1189 as disclosed herein, such as a pharmaceutically acceptable salt of AP1189 selected from the group consisting of AP1189 acetate and AP1189 succinate compared to an alkaline suspension comprising said pharmaceutically acceptable salt of AP1189 is 0.7 to 1.8, such as 0.7, such as 0.8, such as 0.9, such as 1.0, such as 1.1, such as 1.2, such as 1.3, such as 1.4, such as 1.5, such as 1.6, such as 1.7, such as 1.8.

In some embodiments the relative bioavailability for the solid oral formulation comprising AP1189 acetate or AP1189 succinate compared to an alkaline suspension comprising AP1189 acetate or AP1189 succinate is 0.7 to 1.8, such as 0.7, such as 0.8, such as 0.9, such as 1.0, such as 1.1, such as 1.2, such as 1.3, such as 1.4, such as 1.5, such as 1.6, such as 1.7, such as 1.8.

In some embodiments the AUC₀₋₂₄ of the solid oral formulation of the present disclosure is not less than 1000 hr*ng/mL, such as not less than 1200 hr*ng/mL, such as not less than 1400 hr*ng/mL, such as not less than 1600 hr*ng/mL, such as not less than 1800 hr*ng/mL, such as not less than 2000 hr*ng/mL, such as not less than 2500 hr*ng/mL, such as not less than 3000 hr*ng/mL, such as not less than 3500 hr*ng/mL, such as not less than 4000 hr*ng/mL, such as not less than 4500 hr*ng/mL, such as not less than 5000 hr*ng/mL, such as not less than 5500 hr*ng/mL, such as not less than 6000 hr*ng/mL, such as not less than 6500 hr*ng/mL, such as not less than 7000 hr*ng/mL, such as not less than 7500 hr*ng/mL, such as not less than 8000 hr*ng/mL after administration of a single dose of a pharmaceutically acceptable salt of AP1189;
such as after administration of a single dose of a pharmaceutically acceptable salt of AP1189 selected from the group consisting of AP1189 acetate and AP1189 succinate; such as after administration of a single dose of AP1189 acetate or AP1189 succinate.

AUC₀₋ₜ is defined herein as the area under the concentration-time curve from time zero (pre-dose) to the time of last quantifiable concentration was calculated using a linear trapezoidal method.

In some embodiments, the geometric mean AUC₀₋ₜ of the solid oral formulation comprising AP1189 acetate is within 80.00% to 125.00% of an AUC₀₋ₜ of 4423.40 (CV 20.8%) h*ng/mL, after administration of a single dose.

In some embodiments, the geometric mean AUC₀₋ₜ of the solid oral formulation comprising AP1189 succinate is within 80.00% to 125.00% of an AUC₀₋ₜ of 3526.23 (CV 23.0%) h*ng/mL, after administration of a single dose.

In some embodiments, the dose-normalized geometric mean AUC₀₋ₜ/D of the solid oral formulation comprising AP1189 acetate is within 80.00% to 125.00% of an AUC₀₋ₜ of 44.23 (CV 20.8%) h*ng/mL, after administration of a single dose.

In some embodiments, the dose-normalized geometric mean AUC₀₋ₜ/D of the solid oral formulation comprising AP1189 succinate is within 80.00% to 125.00% of an AUC₀₋ₜ of 35.26 (CV 23.0%) h*ng/mL, after administration of a single dose.

In some embodiments, AUC₀₋ₜ is 0-24 hours.

AUC_{0-infinity} AUC_{0-∞} is defined herein as AUC₀₋ₜ + AUC_{t-∞}, where AUC_{t-∞} = Ct/kₑ, where Ct was the measured concentration at time of the last quantifiable concentration t, and kₑ is the terminal plasma elimination rate-constant.

In some embodiments, the geometric mean AUC_{0-infinity} of the solid oral formulation comprising AP1189 acetate is within 80.00% to 125.00% of an AUC_{0-infinity} of 4840.61 (CV 18.2%) h*ng/mL, after administration of a single dose.

In some embodiments, the geometric mean AUC_{0-infinity} of the solid oral formulation comprising AP1189 succinate is within 80.00% to 125.00% of an AUC_{0-infinity} of 4013.18 (CV 20.2%) h*ng/mL, after administration of a single dose.

In some embodiments, the dose-normalized geometric mean AUC_{0-infinity}/D of the solid oral formulation comprising AP1189 acetate is within 80.00% to 125.00% of an AUC_{0-infinity} of 48.41 (CV 18.2%) h*ng/mL, after administration of a single dose.

In some embodiments, the dose-normalized geometric mean AUC_{0-infinity}/D of the solid oral formulation comprising AP1189 succinate is within 80.00% to 125.00% of an AUC_{0-infinity} of 40.13 (CV 20.2%) h*ng/mL, after administration of a single dose.

In some embodiments said AUC is measured after administration of a single dose of 100 mg AP1189 acetate (calculated as free base). In some embodiments said AUC is measured after administration of a single dose of 50 mg AP1189 acetate (calculated as free base). In some embodiments said AUC is measured after administration of a single dose of 200 mg AP1189 acetate (calculated as free base). In some embodiments said AUC is measured after administration of a single dose of 83 mg AP1189 acetate (calculated as free base).

In some embodiments said AUC is measured after administration of a single dose of 100 mg AP1189 succinate (calculated as free base). In some embodiments said AUC is measured after administration of a single dose of 50 mg AP1189 succinate (calculated as free base). In some embodiments said AUC is measured after administration of a single dose of 200 mg AP1189 succinate (calculated as free base).

In some embodiments said AUC is measured after administration of a single dose to a human subject, such as a healthy male Caucasian subject.

In some embodiments said AUC is measured after administration of a single dose to a human subject, such as a healthy male Caucasian subject, in fasting conditions.

In some embodiments, fasting conditions encompass fasting overnight for a minimum of 10 hrs.

In some embodiments said AUC is measured after administration of a single dose to a human subject, such as a healthy male Caucasian subject, in fed conditions.

In some embodiments, fed conditions encompass administering a high-fat standard breakfast 30 min before dosing and after collection of the PK pre-dose blood sample.

In some embodiments the Cₘₐₓ of the solid oral formulation is not less than 180 ng/mL, such as not less than 200 ng/mL, such as not less than 225 ng/mL, such as not less than 250 ng/mL, such as not less than 275 ng/mL, such as not less than 300 ng/mL, such as not less than 350 ng/mL, such as not less than 400 ng/mL, such as not less than 450 ng/mL, such as not less than 500 ng/mL, such as not less than 600 ng/mL, such as not less than 700 ng/mL, such as not less than 800 ng/mL, such as not less than 900 ng/mL, such as not less than 1000 ng/mL, such as not less than 1200 ng/mL, such as not less than 1400 ng/mL, such as not less than 1600 ng/mL, such as not less than 1800 ng/mL, such as not less than 2000 ng/mL, such as not less than 2200 ng/mL, such as not less than 2400 ng/mL, such as not less than 2600 ng/mL, such as not less than 2800 ng/mL, such as not less than 3000 ng/mL, such as not less than 3200 ng/mL, such as not less than 3400 ng/mL, such as not less than 3600 ng/mL, after administration of a single dose of a pharmaceutically acceptable salt of AP1189; such as after administration of a single dose of a pharmaceutically acceptable salt of AP1189 selected from the group consisting of AP1189 acetate and AP1189 succinate; such as after administration of a single dose of AP1189 acetate or AP1189 succinate.

In some embodiments, the geometric mean Cₘₐₓ of the solid oral formulation comprising AP1189 acetate is within 80.00% to 125.00% of **a** Cₘₐₓ of 302.70 (CV 31.1%) ng/mL, after administration of a single dose.

In some embodiments, the geometric mean Cₘₐₓ of the solid oral formulation comprising AP1189 succinate is within 80.00% to 125.00% of **a** Cₘₐₓ of 255.93 (CV 18.6%) ng/mL, after administration of a single dose.

In some embodiments, the dose-normalized geometric mean Cₘₐₓ/D of the solid oral formulation comprising AP1189 acetate is within 80.00% to 125.00% of a Cₘₐₓ/D of 3.03 (CV 31.1%) ng/mL/mg, after administration of a single dose.

In some embodiments, the dose-normalized geometric mean Cₘₐₓ/D of the solid oral formulation comprising AP1189 succinate is within 80.00% to 125.00% of a Cₘₐₓ/D of 2.56 (CV 18.6%) ng/mL/mg, after administration of a single dose.

In some embodiments said AUC is measured after administration of a single dose of 100 mg AP1189 acetate (calculated as free base). In some embodiments said AUC is measured after administration of a single dose of 50 mg AP1189 acetate (calculated as free base). In some embodiments said AUC is measured after administration of a single dose of 200 mg AP1189 acetate (calculated as free base). In some embodiments said AUC is measured after administration of a single dose of 83 mg AP1189 acetate (calculated as free base).

In some embodiments said AUC is measured after administration of a single dose of 100 mg AP1189 succinate (calculated as free base). In some embodiments said AUC is measured after administration of a single dose of 50 mg AP1189 succinate (calculated as free base). In some embodiments said AUC is measured after administration of a single dose of 200 mg AP1189 succinate (calculated as free base).

In some embodiments said Cₘₐₓ is measured after administration of a single dose to a human subject, such as a healthy male Caucasian subject.

In some embodiments said Cₘₐₓ is measured after administration of a single dose to a human subject, such as a healthy male Caucasian subject, in fasting conditions.

In some embodiments said Cₘₐₓ is measured after administration of a single dose to a human subject, such as a healthy male Caucasian subject, in fed conditions.

In some embodiments the solid oral dosage form comprises a pharmaceutically acceptable salt of AP1189 at a dosage from 25 mg to 650 mg per tablet, such as 25 mg, such as 50 mg, such as 100 mg, such as 150 mg, such as 200 mg, such as 250 mg, such as 300 mg, such as 350 mg, such as 400 mg, such as 450 mg, such as 500 mg, such as 550 mg, such as 600 mg, such as 650 mg AP1189 (calculated as AP1189 free base).

In some embodiments the solid oral dosage form comprises a pharmaceutically acceptable salt of AP1189 selected from the group consisting of AP1189 acetate and AP1189 succinate at a dosage from 25 mg to 650 mg per tablet, such as 25 mg, such as 50 mg, such as 100 mg, such as 150 mg, such as 200 mg, such as 250 mg, such as 300 mg, such as 350 mg, such as 400 mg, such as 450 mg, such as 500 mg, such as 550 mg, such as 600 mg, such as 650 mg AP1189 (calculated as AP1189 free base).

In some embodiments the solid oral dosage form comprises AP1189 acetate at a dosage from 25 mg to 650 mg per tablet, such as 25 mg, such as 50 mg, such as 100 mg, such as 150 mg, such as 200 mg, such as 250 mg, such as 300 mg, such as 350 mg, such as 400 mg, such as 450 mg, such as 500 mg, such as 550 mg, such as 600 mg, such as 650 mg AP1189 (calculated as AP1189 free base). For the sake of clarity, a tablet comprising 100 mg AP1189 will comprise 120 mg AP1189 acetate.

In some embodiments the solid oral dosage form comprises AP1189 acetate at a dosage of 25 mg per tablet, such as 50 mg, such as 100 mg, such as 150 mg, such as 200 mg per tablet.

In some embodiments the solid oral dosage form comprises AP1189 succinate at a dosage from 25 mg to 650 mg per tablet, such as 25 mg, such as 50 mg, such as 100 mg, such as 150 mg, such as 200 mg, such as 250 mg, such as 300 mg, such as 350 mg, such as 400 mg, such as 450 mg, such as 500 mg, such as 550 mg, such as 600 mg, such as 650 mg AP1189 (calculated as AP1189 free base). For the sake of clarity, a tablet comprising 100 mg AP1189 will comprise 140 mg AP1189 succinate.

In some embodiments the solid oral dosage form comprises AP1189 succinate at a dosage of 25 mg per tablet, such as 50 mg, such as 100 mg, such as 150 mg, such as 200 mg per tablet.

It is an aspect of the present disclosure to provide a solid oral formulation comprising a pharmaceutically acceptable salt of AP1189 selected from the group consisting of AP1189 acetate and AP1189 succinate, and at least one pharmaceutically acceptable excipient;
such as one or more gastroretentive excipients; such as one or more immediate release excipients.

It is an aspect of the present disclosure to provide a solid oral formulation comprising (*E*)-*N*-[1-(2-nitrophenyl)-1-*H*-pyrrole-2-yl-allylideneamino]-guanidinium acetate (AP1189 acetate), and at least one pharmaceutically acceptable excipient.

It is an aspect of the present disclosure to provide a solid oral formulation comprising (*E*)-*N*-[1-(2-nitrophenyl)-1-*H*-pyrrole-2-yl-allylideneamino]-guanidinium acetate (AP1189 acetate), and at least one pharmaceutically acceptable excipient, such as one or more gastroretentive excipients.

It is an aspect of the present disclosure to provide a solid oral formulation comprising (*E*)-*N*-[1-(2-nitrophenyl)-1-*H*-pyrrole-2-yl-allylideneamino]-guanidinium acetate (AP1189 acetate), and at least one pharmaceutically acceptable excipient, such as one or more immediate release excipients.

It is an aspect of the present disclosure to provide a solid oral formulation comprising (*E*)-*N*-[1-(2-nitrophenyl)-1-*H*-pyrrole-2-yl-allylideneamino]-guanidinium succinate (AP1189 succinate), and at least one pharmaceutically acceptable excipient, such as one or more immediate release excipients.

The solid oral formulation, such as the immediate release solid oral formulation of the present disclosure may be formulated by any means know to the skilled person to provide immediate release of said pharmaceutically acceptable salt of AP1189.

The solid oral formulation, such as the immediate release solid oral formulation of the present disclosure may be formulated by any means know to the skilled person to provide immediate release of said pharmaceutically acceptable salt of AP1189 in the gastric compartment.

In some embodiments the oral formulation, such as solid oral formulation, of the present disclosure, comprises said at least one pharmaceutically acceptable excipient.

In some embodiments said at least one pharmaceutically acceptable excipient comprises a filler. In some embodiments said at least one pharmaceutically acceptable excipient comprises one or more fillers.

In some embodiments said at least one pharmaceutically acceptable excipient comprises a filler which is microcrystalline cellulose (MCC).

In some embodiments said at least one pharmaceutically acceptable excipient comprises a filler selected from the group consisting of microcrystalline cellulose (MCC), silicified microcrystalline cellulose and mannitol.

In some embodiments said at least one pharmaceutically acceptable excipient comprises one or more binders.

In some embodiments said at least one pharmaceutically acceptable excipient comprises one or more binders which is maltodextrin.

In some embodiments said at least one pharmaceutically acceptable excipient comprises one or more disintegrants.

In some embodiments said at least one pharmaceutically acceptable excipient comprises one or more intergranular disintegrants.

In some embodiments said at least one pharmaceutically acceptable excipient comprises one or more disintegrants which is sodium starch glycolate.

In some embodiments said at least one pharmaceutically acceptable excipient comprises one or more wetting agents.

In some embodiments said at least one pharmaceutically acceptable excipient comprises one or more wetting agents which is polysorbate 80.

In some embodiments said at least one pharmaceutically acceptable excipient comprises one or more flow agents or lubricants.

In some embodiments said at least one pharmaceutically acceptable excipient comprises one or more flow agents or lubricants which is magnesium stearate.

In some embodiments the oral formulation is first made as a granulate comprising said pharmaceutically acceptable salt of AP1189, which granulate is subsequently compressed into tablets.

In some embodiments granulate is a wet granulate.

In some embodiments the oral formulation is first made as a granulate comprising said pharmaceutically acceptable salt of AP1189 and one or more pharmaceutically acceptable excipients, which granulate is subsequently compressed into tablets.

In some embodiments the oral formulation is first made as a granulate comprising said pharmaceutically acceptable salt of AP1189 and one or more pharmaceutically acceptable excipients, which granulate is further mixed with one or more further pharmaceutically acceptable excipients (or fillers) and subsequently compressed into tablets.

In some embodiments the granulate comprises from 30% to 70% w/w AP1189 (free base); such as 30 to 35% w/w, such as 35 to 40% w/w such as 40 to 45% w/w, such as 45 to 50% w/w such as 50 to 55% w/w, such as 55 to 60% w/w such as 60 to 65% w/w, such as 65 to 70% w/w such as 60 to 65% w/w, such as 65 to 70% w/w of AP1189 (free base).

In some embodiments the granulate comprises 50% w/w, such as 55% w/w, such as 60% w/w, such as 65% w/w, such as 70% w/w, such as 75% w/w of AP1189 (free base).

In some embodiments the granulate comprises from 60% to 90% w/w of the pharmaceutically acceptable salt of AP1189, such as 65% to 85% w/w of the pharmaceutically acceptable salt of AP1189; such as 60 to 65% w/w, such as 65 to 70% w/w, such as 70 to 75% w/w, such as 70 to 75% w/w, such as 75 to 80% w/w, such as 80 to 85% w/w, such as 85 to 90% w/w of the pharmaceutically acceptable salt of AP1189; such as of a pharmaceutically acceptable salt of AP1189 selected from the group consisting of AP1189 acetate and AP1189 succinate; such as AP1189 acetate or AP1189 succinate.

In some embodiments the granulate comprises 60% w/w, such as 65% w/w, such as 70% w/w, such as 75% w/w, such as 80% w/w, such as 85% w/w, such as 90% w/w of the pharmaceutically acceptable salt of AP1189; such as of a pharmaceutically acceptable salt of AP1189 selected from the group consisting of AP1189 acetate and AP1189 succinate; such as AP1189 acetate or AP1189 succinate.

In some embodiments the granulate comprises 75% w/w of a pharmaceutically acceptable salt of AP1189 selected from the group consisting of AP1189 acetate and AP1189 succinate.

In some embodiments the granulate comprises 75% w/w of AP1189 acetate.

In some embodiments the granulate comprises 75% w/w AP1189 succinate.

In some embodiments the granulate comprises 62.4% w/w AP1189 (free base).

In some embodiments the granulate comprises said at least one pharmaceutically acceptable excipient.

In some embodiments the granulate comprises at least one pharmaceutically acceptable excipient selected from the group consisting of a filler, a binder, a disintegrant and a wetting agent.

In some embodiments the granulate comprises a pharmaceutically acceptable salt of AP1189, such as a pharmaceutically acceptable salt of AP1189 selected from the group consisting of AP1189 acetate and AP1189 succinate, and at least one pharmaceutically acceptable excipient selected from the group consisting of a filler, a binder, a disintegrant and a wetting agent.

In some embodiments the granulate comprises AP1189 acetate or AP1189 succinate, and at least one pharmaceutically acceptable excipient selected from the group consisting of a filler, a binder, a disintegrant and a wetting agent.

In some embodiments the granulate comprises a filler, a binder, a disintegrant and a wetting agent.

In some embodiments the granulate is mixed with one or more additional fillers.

In some embodiments the granulate is mixed with one or more additional fillers prior to compression into a tablet.

In some embodiments the granulate is mixed with one or more additional fillers and one or more flow agents or lubricants.

In some embodiments the granulate is mixed with one or more additional fillers and one or more flow agents or lubricants prior to compression into a tablet.

In some embodiments the one or more additional fillers are selected from the group consisting of silicified microcrystalline cellulose and mannitol.

In some embodiments the granulate is mixed with the fillers silicified microcrystalline cellulose and mannitol at a ratio of 3:1.

In some embodiments the one or more flow agents or lubricants is magnesium stearate.

In some embodiments the granulate is compressed into a tablet.

In some embodiments the tablet thickness is 1 to 20 mm, such as 1 to 2 mm, such as 2 to 3 mm, such as 3 to 4 mm, such as 4 to 5 mm, such as 5 to 6 mm, such as 6 to 7 mm, such as 7 to 8 mm, such as 8 to 9 mm, such as 9 to 10 mm, such as 10 to 11 mm, such as 11 to 12 mm, such as 12 to 13 mm, such as 13 to 14 mm, such as 14 to 15 mm, such as 15 to 16 mm, such as 16 to 17 mm, such as 17 to 18 mm, such as 18 to 19 mm, such as 19 to 20 mm.

In some embodiments the tablet thickness is 2 mm, such as 2.5 mm, such as 3 mm, such as 3.5 mm, such as 4 mm, such as 4.5 mm, such as 5 mm, such as 5.5 mm, such as 6 mm, such as 6.5 mm, such as 7 mm, such as 7.5 mm, such as 8 mm, such as 8.5 mm, such as 9 mm.

In some embodiments the tablet thickness is 5.5 mm.

In some embodiments the tablet is an oval shape.

In some embodiments the tablet is compressed into an oval shape. In some embodiments the tablet is an oval shape.

In some embodiments the tablet is 15.5 x 7.1 mm. In some embodiments the tablet is 10 to 20 mm x 5 to 10 mm.

In some embodiments the tablet is coated. In some embodiments the tablet is film coated.

In some embodiments the tablet is coated with hypromellose.

In some embodiments the tablet is coated to achieve a weight gain of 4%. In some embodiments the tablet is coated to achieve a weight gain of 2 to 6%, such as 2 to 3%, such as 3 to 4%, such as 4 to 5%, such as 5 to 6%.

In some embodiments the tablet is coated to achieve a weight gain of 1%, such as of 2%, such as of 3%, such as of 4%, such as of 5%, such as of 6%, such as of 7%, such as of 8%, such as of 9%, such as of 10%.

### Dosage

According to the present disclosure, the oral formulation comprising a compound of the disclosure is administered to individuals in need of treatment in pharmaceutically effective doses. A therapeutically effective amount of a compound is an amount sufficient to cure, prevent, reduce the risk of, alleviate or partially arrest the clinical manifestations of a given disease and its complications. The amount that is effective for a particular therapeutic purpose will depend on the severity and the sort of the disorder as well as on the weight and general state of the subject. The compound may be administered one or several times per day, such as from 1 to 8 times per day, such as from 1 to 6 times per day, such as from 1 to 5 times per day, such as from 1 to 4 times per day, such as from 1 to 3 times per day, such as from 1 to 2 times per day, such as from 2 to 4 times per day, such as from 2 to 3 times per day. Alternatively, the compound may be administered less than once a day, for example once a day, such as once every second day, for example once every third day, such as once every fourth day, for example once every fifth day, such as once every sixth day, for example once every week.

In some embodiments the oral formulation comprising a compound of the disclosure is administered in a therapeutically effective amount, such as in an amount of 1 mg to 1000 mg AP1189 per day (calculated as the free base).

It follows that in some embodiments the pharmaceutically acceptable salt of AP1189 is administered in an amount of 1 mg to 1000 mg, such as 1 to 5 mg, 5 to 10 mg, 10 to 15 mg, 15 to 20 mg, 20 to 25 mg, 25 to 50 mg, 50 to 75 mg, 75 to 100 mg, 100 to 125 mg, 125 to 150 mg, 150 to 175 mg, 175 to 200 mg, 200 to 250 mg, 250 to 300 mg, 300 to 350 mg, 350 to 400 mg, 400 to 450 mg, 450 to 500 mg, 500 to 600 mg, 600 to 700 mg, 700 to 800 mg, 800 to 900 mg, or 900 to 1000 mg per day. All dosages of the pharmaceutically acceptable salts of AP1189 indicated herein are calculated as the free base, unless expressly indicated otherwise.

In some embodiments, 'per day' means the dosage is given in one dosage or is divided in multiple dosages per day, including once a day (QD), twice a day (BID) and/or three times a day (TID).

In some embodiments the oral formulation of the present disclosure is administered once daily.

In some embodiments the pharmaceutically acceptable salt of AP1189 is administered in an amount of 1 mg to 1000 mg, such as 1 to 5 mg, 5 to 10 mg, 10 to 15 mg, 15 to 20 mg, 20 to 25 mg, 25 to 50 mg, 50 to 75 mg, 75 to 100 mg, 100 to 125 mg, 125 to 150 mg, 150 to 175 mg, 175 to 200 mg, 200 to 250 mg, 250 to 300 mg, 300 to 350 mg, 350 to 400 mg, 400 to 450 mg, 450 to 500 mg, 500 to 600 mg, 600 to 700 mg, 700 to 800 mg, 800 to 900 mg, or 900 to 1000 mg per dosage.

In some embodiments the pharmaceutically acceptable salt of AP1189 is administered in an amount of 25 mg once daily, 50 mg once daily, 100 mg once daily, 200 mg once daily, 300 mg once daily, 400 mg once daily, 500 mg once daily, 600 mg once daily, 700 mg once daily, 800 mg once daily, 900 mg once daily or 1000 mg once daily.

In some embodiments the pharmaceutically acceptable salt of AP1189 is administered in an amount of 50 mg once daily.

In some embodiments the pharmaceutically acceptable salt of AP1189 is administered in an amount of 50 mg two times daily (BID) or 50 mg three times daily (TID).

In some embodiments the pharmaceutically acceptable salt of AP1189 is administered in an amount of 100 mg once daily.

In some embodiments the pharmaceutically acceptable salt of AP1189 is administered in an amount of 100 mg two times daily (BID) or 100 mg three times daily (TID).

In some embodiments the pharmaceutically acceptable salt of AP1189 is administered in an amount of 200 mg once daily.

In some embodiments the pharmaceutically acceptable salt of AP1189 is administered in an amount of 200 mg two times daily (BID) or 200 mg three times daily (TID).

In some embodiments AP1189, or a pharmaceutically acceptable salt thereof, is administered in an amount of 400 mg once daily.

In some embodiments the pharmaceutically acceptable salt of AP1189 is administered in an amount of 400 mg two times daily (BID) or 200 mg three times daily (TID).

In some embodiments a daily dosage of a pharmaceutically acceptable salt of AP1189 is split into multiple dosage forms and/or administrations.

In another embodiment the pharmaceutically acceptable salt of AP1189 is administered in an amount of 0.1 to 0.5 mg/kg bodyweight, such as 0.5 mg to 1 mg/kg bodyweight, such as 1 to 2 mg/kg bodyweight, such as 2 to 3 mg/kg bodyweight, such as 3 to 5 mg/kg bodyweight, such as 5 to 10 mg/kg bodyweight, such as 10 to 15 mg/kg bodyweight, such as 15 to 20 mg/kg bodyweight, such as 20 to 25 mg/kg bodyweight, for example 25 to 30 mg/kg bodyweight.

### Medical use

It is an aspect of the present disclosure to provide an oral formulation as disclosed herein comprising a pharmaceutically acceptable salt of AP1189 disclosed herein as soluble or highly soluble at pH 1.2, such as a pharmaceutically acceptable salt of AP1189 selected from the group consisting of AP1189 acetate and AP1189 succinate, in particular AP1189 acetate or AP1189 succinate, for use in the treatment of a disease or disorder.

It is an aspect of the present disclosure to provide an oral formulation as disclosed herein comprising a pharmaceutically acceptable salt of AP1189 disclosed herein as soluble or highly soluble at pH 1.2, for use in the treatment of a disease or disorder in a subject, wherein the subject to be treated is a mammal. In some embodiment the mammal is a human being. In some embodiments the mammal is a domestic animal. In some embodiments the mammal is selected from the group consisting of mice, rats, dogs, cats, horses, cows, sheep and pigs.

In some embodiments, the disease or disorder is selected from the group consisting of a kidney disease, an arthritic disease, a viral disease or disorder, a cardiovascular disease and/or atherosclerosis, and systemic inflammatory disorders.

The references to methods of treatment in this description are to be interpreted as references to the compounds, pharmaceutical compositions and medicaments of the present invention for use in a method for the treatment of the human (or animal) body by therapy (or for diagnosis)

### Kidney disease

It is as aspect of the present disclosure to provide an oral formulation, a pharmaceutical composition, or unit dosage form according to the present disclosure for use in treating or preventing a kidney disease.

Also disclosed is a method of treating or preventing a kidney disease in a subject in need thereof, wherein the subject is administered a therapeutically effect amount of the oral formulation, pharmaceutical composition, or unit dosage form of the present disclosure.

Also disclosed is the use of an oral formulation, pharmaceutical composition, or unit dosage form according to the present disclosure for use in the manufacture of a medicament for the treatment or prevention of a kidney disease.

In some embodiments of the present disclosure there is provided an oral formulation, such as a solid oral formulation, comprising a pharmaceutically acceptable salt of AP1189 as disclosed herein, such as AP1189 acetate or AP1189 succinate, and at least one pharmaceutically acceptable excipient, as disclosed herein, for use in treating or preventing a kidney disease.

In some embodiments said kidney disease present with proteinuria. In some embodiments said kidney disease is a proteinuric kidney disease.

In some embodiments said kidney disease is a a glomerular disease

In some embodiments said kidney disease is nephrotic syndrome (glomerulonephrosis).

In some embodiments said kidney disease primary nephrotic syndrome (primary glomerulonephrosis).

In some embodiments said primary nephrotic syndrome is membranous glomerulonephritis (MGN) (or membranous nephropathy (MN)).

In some embodiments said primary nephrotic syndrome is focal segmental glomerulosclerosis (FSGS).

In some embodiments said primary nephrotic syndrome is membranoproliferative glomerulonephritis (MPGN) (mesangiocapillary glomerulonephritis).

In some embodiments said membranoproliferative glomerulonephritis (MPGN) is selected from Type 1 MPGN and Type 2 MPGN.

In some embodiments said primary nephrotic syndrome is rapidly progressive glomerulonephritis (RPGN) (crescentic GN).

In some embodiments said primary nephrotic syndrome is minimal change disease (MCD).

In some embodiments said kidney disease is secondary nephrotic syndrome (secondary glomerulonephrosis).

In some embodiments said said secondary nephrotic syndrome is caused by an underlying autoimmune disease, an underlying cancer disease, an underlying genetic disorder, or by an underlying disease selected from the group consisting of: Systemic lupus erythematosus (SLE), Diabetic nephropathy, Sarcoidosis, Sjögren's syndrome, Amyloidosis, Multiple myeloma, Vasculitis, Cancer and Genetic disorders (such as congenital nephrotic syndrome).

In some embodiments said secondary nephrotic syndrome is caused by Diabetic nephropathy, by an infection, such as a urinary tract infection, such as an infection selected from the group consisting of HIV, syphilis, hepatitis such as hepatitis A, B and C, post-streptococcal infection, urinary schistosomiasis and Ebola. In some embodiments said secondary nephrotic syndrome is drug-induced
In some embodiments said kidney disease is an inflammatory kidney disease.

In some embodiments said kidney disease is glomerulonephritis (GN). In some embodiments said glomerulonephritis is selected from the group consisting of IgA nephropathy (Berger's disease), IgM nephropathy, Post-infectious glomerulonephritis and Thin basement membrane disease.

In some embodiment said kidney disease is idiopathic membranous nephropathy (iMN).

In some embodiments there is provided an oral formulation, a pharmaceutical composition, or unit dosage form according to the present disclosure for use in treating or preventing idiopathic membranous nephropathy (iMN).

### Arthritic disease

It is as aspect of the present disclosure to provide an oral formulation, a pharmaceutical composition, or unit dosage form according to the present disclosure for use in treating or preventing an arthritic disease.

Also disclosed is a method of treating or preventing an arthritic disease in a subject in need thereof, wherein the subject is administered a therapeutically effect amount of the oral formulation, pharmaceutical composition, or unit dosage form of the present disclosure.

Also disclosed is the use of an oral formulation, pharmaceutical composition, or unit dosage form according to the present disclosure for use in the manufacture of a medicament for the treatment or prevention of an arthritic disease.

In some embodiments there is provided an oral formulation as disclosed herein comprising a pharmaceutically acceptable salt of AP1189 disclosed herein as soluble or highly soluble at pH 1.2, for use in the treatment of an arthritic disease in a subject, wherein the subject to be treated is a mammal. In some embodiment the mammal is a human being. In some embodiments the mammal is a domestic animal. In some embodiments the mammal is selected from the group consisting of mice, rats, dogs, cats, horses, cows, sheep and pigs.

In some embodiments of the present disclosure there is provided an oral formulation, such as a solid oral formulation, comprising a pharmaceutically acceptable salt of AP1189 as disclosed herein, such as AP1189 acetate or AP1189 succinate, and at least one pharmaceutically acceptable excipient, as disclosed herein, for use in treating or preventing an arthritic disease.

In one embodiment the arthritic disease is an auto-immune disease and/or an inflammatory disease that presents with joint inflammation.

In one embodiment, the arthritic disease is selected from the group consisting of inflammatory arthritis, degenerative arthritis, metabolic arthritis, reactive arthritis and infectious arthritis.

In one embodiment, the arthritic disease is inflammatory arthritis.

In one embodiment, the inflammatory arthritis is selected from the group consisting of Rheumatoid Arthritis (RA), Psoriatic Arthritis, and Ankylosing Spondylitis.

In one embodiment, the inflammatory arthritis is Rheumatoid Arthritis (RA).

In one embodiment, the rheumatoid arthritis is severe active RA (CDAI > 22). In one embodiment, the rheumatoid arthritis is RA with a CDAI > 22.

In one embodiment, the rheumatoid arthritis is RA with a DAS28 score of above 5.1.

In one embodiment, the rheumatoid arthritis is juvenile rheumatoid arthritis (JRA)

In one embodiment, the degenerative arthritis is osteoarthritis.

In one embodiment, the metabolic arthritis is gouty arthritis.

In one embodiment, the reactive and/or infectious arthritis is arthritis associated with infection with one or more of Hepatitis C, Chlamydia, gonorrhoea, salmonella or shigella.

In one embodiment the arthritic disease is arthritis as part of a systemic inflammatory disease.

In one embodiment, the arthritis as part of a systemic inflammatory disease, such as an inflammatory disease selected from the group consisting of Systemic lupus erythematosus, mixed connective tissue disease, Still's disease, and Polymyalgia Rheumatica.

In some embodiments there is provided an oral formulation, a pharmaceutical composition, or unit dosage form according to the present disclosure for use in treating or preventing rheumatoid arthritis.

In some embodiments there is provided an oral formulation, a pharmaceutical composition, or unit dosage form according to the present disclosure in combination with MTX (methotrexate) for use in treating or preventing rheumatoid arthritis.

In some embodiments there is provided an oral formulation, a pharmaceutical composition, or unit dosage form according to the present disclosure, alone or in combination with MTX (methotrexate), for use in treating or preventing rheumatoid arthritis in patients with an inappropriate response to MTX (such as patients with a reduced response to MTX treatment, such as an MTX non-responder).

### Viral disease or disorder

It is as aspect of the present disclosure to provide an oral formulation, a pharmaceutical composition, or unit dosage form according to the present disclosure for use in treating or preventing a viral disease or disorder.

Also disclosed is a method of treating or preventing a viral disease or disorder in a subject in need thereof, wherein the subject is administered a therapeutically effect amount of the oral formulation, pharmaceutical composition, or unit dosage form of the present disclosure.

Also disclosed is the use of an oral formulation, pharmaceutical composition, or unit dosage form according to the present disclosure for use in the manufacture of a medicament for the treatment or prevention of a viral disease or disorder.

In some embodiments of the present disclosure there is provided an oral formulation, such as a solid oral formulation, comprising a pharmaceutically acceptable salt of AP1189 as disclosed herein, such as AP1189 acetate or AP1189 succinate, and at least one pharmaceutically acceptable excipient, as disclosed herein, for use in treating or preventing a viral disease or disorder.

In some embodiments said viral disease or disorder is a symptomatic viral disease or disorder.

In some embodiments said viral disease or disorder is a symptomatic viral disease or disorder with inflammation, such as hyperinflammation.

In some embodiments said viral disease or disorder is a symptomatic viral disease or disorder with inflammation, such as hyperinflammation, in one or more organs. Inflammation in one or more organs may also be referred to as local inflammation.

In some embodiments said one or more organs are selected from the group consisting of lungs, the respiratory tract, kidney, liver, pancreas, spleen, exocrine glands, endocrine glands, lymph nodes, brain, heart, muscles, bone marrow, skin, skeleton, bladder, reproduction organs including the phallopian tubes, eye, ear, vascular system, the gastroinstestinal tract including small intestines, colon, rectum, canalis analis and the prostate gland.

In some embodiments said viral disease or disorder is inflammatory viral diseases or disorders.

In some embodiments said viral disease or disorder is a viral respiratory infection, such as a viral lower respiratory infection.

In some embodiments said viral disease or disorder is viral respiratory diseases or disorders.

In some embodiments said viral disease or disorder is viral diseases or disorders of the lung.

In some embodiments said viral disease or disorder is viral diseases or disorders with inflammation in the respiratory system, such as in the lungs and/or respiratory tract.

In some embodiments said viral disease or disorder is viral diseases or disorders with one or more respiratory symptoms. In one embodiment said one or more respiratory symptoms are selected from the group consisting of cough, dry cough, dyspnea, impaired oxygenation, respiratory illness, respiratory dysfunction, respiratory failure, respiratory syndrome and acute respiratory disease (ARD).

In some embodiments said viral disease or disorder is severe disease. Severe disease present with dyspnoea, increased respiratory frequency, reduced blood oxygen saturation and/or lung infiltrates.

In some embodiments said viral disease or disorder is critical disease. Critical disease present with respiratory failure, septic shock, and/or multiple organ dysfunction (MOD) or multiple organ failure (MOF).

In some embodiments said viral disease or disorder is viral pneumonia.

In some embodiments said viral disease or disorder is viral bronchiolitis.

In some embodiments said viral disease or disorder is viral diseases or disorders with respiratory failure.

In some embodiments said viral disease or disorder is acute respiratory distress syndrome (ARDS).

In some embodiments said viral disease or disorder is viral acute respiratory distress syndrome (ARDS), such as virally-induced ARDS.

In some embodiments said viral disease or disorder is symptomatic COVID-19 with acute respiratory distress syndrome (ARDS).

In some embodiments there is provided an oral formulation, a pharmaceutical composition, or unit dosage form according to the present disclosure for use in treating or preventing ARDS, such as viral ARDS.

In some embodiments said viral disease or disorder is viral diseases and disorders with systemic inflammatory distress syndrome (SIDS) and/or sepsis.

In some embodiments said viral disease or disorder is viral diseases and disorders with pulmonary insufficiency.

In some embodiments said viral disease or disorder is viral diseases or disorders with cytokine release syndrome (CRS) and/or a cytokine storm (hypercytokinemia).

In some embodiments said viral disease or disorder is caused by a viral infection selected from the group consisting of Severe Acute Respiratory Syndrome CoronaVirus 2 (SARS-CoV-2), often referred to as the COVID-19 virus; SARS-CoV, MERS-CoV, the dengue virus and influenza virus (including Types A, B and C).

### Cardiovascular disease and/or atherosclerosis

It is as aspect of the present disclosure to provide an oral formulation, a pharmaceutical composition, or unit dosage form according to the present disclosure for use in treating or preventing a cardiovascular disease and/or atherosclerosis.

Also disclosed is a method of treating or preventing a cardiovascular disease and/or atherosclerosis in a subject in need thereof, wherein the subject is administered a therapeutically effect amount of the oral formulation, pharmaceutical composition, or unit dosage form of the present disclosure.

Also disclosed is the use of an oral formulation, pharmaceutical composition, or unit dosage form according to the present disclosure for use in the manufacture of a medicament for the treatment or prevention of a cardiovascular disease and/or atherosclerosis.

In some embodiments of the present disclosure there is provided an oral formulation, such as a solid oral formulation, comprising a pharmaceutically acceptable salt of AP1189 as disclosed herein, such as AP1189 acetate or AP1189 succinate, and at least one pharmaceutically acceptable excipient, as disclosed herein, for use in treating or preventing a cardiovascular disease and/or atherosclerosis.

In some embodiments said cardiovascular disease is selected from the group consisting of coronary artery diseases (CAD) such as angina and myocardial infarction (commonly known as a heart attack); stroke, heart failure, hypertensive heart disease, rheumatic heart disease, cardiomyopathy, abnormal heart rhythms, congenital heart disease, valvular heart disease, carditis, aortic aneurysms, peripheral artery disease, vascular disease, thromboembolic disease, and venous thrombosis.

In some embodiments said cardiovascular disease is atherosclerotic cardiovascular disease.

In some embodiments said atherosclerotic cardiovascular disease is selected from the group consisting of coronary artery disease, stroke (cerebrovascular disease), and peripheral artery disease.

In some embodiments said cardiovascular disease is vascular inflammation.

### Systemic inflammatory disorders

It is as aspect of the present disclosure to provide an oral formulation, a pharmaceutical composition, or unit dosage form according to the present disclosure for use in treating or preventing a systemic inflammatory disorder.

Also disclosed is a method of treating or preventing a systemic inflammatory disorder in a subject in need thereof, wherein the subject is administered a therapeutically effect amount of the oral formulation, pharmaceutical composition, or unit dosage form of the present disclosure.

Also disclosed is the use of an oral formulation, pharmaceutical composition, or unit dosage form according to the present disclosure for use in the manufacture of a medicament for the treatment or prevention of a systemic inflammatory disorder.

In some embodiments of the present disclosure there is provided an oral formulation, such as a solid oral formulation, comprising a pharmaceutically acceptable salt of AP1189 as disclosed herein, such as AP1189 acetate or AP1189 succinate, and at least one pharmaceutically acceptable excipient, as disclosed herein, for use in treating or preventing a systemic inflammatory disorder.

Systemic disorders with possible involvement of the nervous system include a variety of diseases with presumed inflammatory and autoimmune pathomechanisms, among them Behçet disease, sarcoidosis, systemic lupus erythematosus, juvenile idiopathic arthritis, scleroderma, and Sjögren syndrome. This disease group encompasses systemic inflammatory disorders with a genetically defined dysregulation of the innate immune system as well as systemic autoimmune disorders characterized by alterations of the adaptive immunity such as autoantibodies and autoreactive T cells.

In some embodiments said systemic inflammatory disorder is an autoimmune disorder. In some embodiments said systemic inflammatory disorder is selected from the group consisting of Behçet disease, sarcoidosis, systemic lupus erythematosus, juvenile idiopathic arthritis, scleroderma, Sjögren syndrome, myositis including dermamyositis and polymyositis, vasculitis, giant cell arteritis, ankylosing spondylitis, polymyalgia rheumatic and psoriatic arthritis.

### Examples

### Example 1: Solubility of AP1189 salts

### Methods

The solubility of AP1189 acetate, tosylate, fumarate, and succinate salts were assessed in 0.2 M buffer solutions having pH of 1.2, 4.5, and 6.8; or in 0.5 M buffer solutions having pH of 1.2 and 4.5. An amount of the salt was suspended in buffer and agitated at ambient temperature for 24 hr, before the supernatant was sampled and analysed for the content of AP1189 by HPLC.

### Exemplary procedure for formation of acetate salt

0.9 equivalent of acetic acid was slowly whilst stirring added to 3-[1-(2-nitrophenyl)-1-H-pyrrole-2-yl]-propanal and aminoguanidine hydrogen carbonate in ethanol. Heated at 50-55 °C for 1 hour before additional 0.11 equivalent acetic acid was added and the mixture was heated to reflux for at least 2 hours. The suspension was cooled to 60 °C before tert-butyl methyl ether was added. Cooled and kept at 2-5 °C for 10-16 hours. Filtered and washed with tert-butyl methyl ether, before recrystallized from ethanol. The procedure produces a polymorph of AP1189 acetate salt corresponding to XRPD pattern 1 ('Form A' in Table 1b).

### Exemplary procedure for formation of succinate salt of AP1189

2-propanol:water 90:10 v/v was added to AP1189 acetate to prepare a slurry. 2-propanol:water 90:10 v/v was added to 1.1 equivalents of succinic acid. The counterion slurry was added to the acetate salt slurry. Temperature cycling was carried out between ambient and 40 °C for ca. 18 h with 4 hour hold periods at ambient temperature and 4 hour hold periods at 40 °C. The entire slurry was then isolated by Büchner filtration and washed with deionised water. The solids were dried under vacuum at ambient.

The procedure produced a polymorph of AP1189 succinate salt corresponding to XRPD pattern 1 ('Form B' in Table 1b)..

The tosylate salt of AP1189 having XRPD pattern 1 was prepared by crystallisation from methanol.

The fumarate salt of AP1189 having XRPD pattern 1 ('Form D' in Table 1b) was prepared by crystallisation from isopropylalcohol:water 90:10 v/v.

### Results

The results of the study are shown in table 1a and Table 1b.

**Table 1a: Thermodynamic solubility results using 0.2 M buffers**

| Test compound | Buffer | Initial pH | 24 h pH | Salt or freebase input concentration (mM) | Solubility at 24 h (mM freebase) |
|---|---|---|---|---|---|
| Acetate salt | pH 1.2 | 3.74 → 1.24 | 3.44 → 1.23 | 210* | 134.10 |
| | pH 4.5 | 4.52 | 4.54 | 74 | 2.23 |
| | pH 6.8 | 6.85 | 6.81 | 69 | 1.18 |
| Tosylate Salt | pH 1.2 | 1.22 | 1.26 | 31 | 0.31 |
| | pH 4.5 | 4.91 → 4.45 | 4.47 | 34 | 0.17 |
| | pH 6.8 | 6.77 | 6.87 | 30 | - ^{‡} |
| Fumarate Salt | pH 1.2 | 1.35 → 1.18 | 1.29 | 38 | 10.90 |
| | pH 4.5 | 4.47 | 4.48 | 39 | 2.73 |
| | pH 6.8 | 6.61 → 11.24 → 6.84 | 6.65 → 6.74 | 39 | 0.86 |
| Succinate Salt | pH 1.2 | 1.62 → 1.21 | 1.36 → 1.26 | 89 * | >36.26** |
| | pH 4.5 | 4.48 | 4.56 | 36 | 4.26 |
| | pH 6.8 | 6.61 → 6.80 | 6.42 → 7.03 → 6.82 | 41 | 0.78 |

| | | | | | |
|---|---|---|---|---|---|
| →: pH adjusted using hydrochloric acid or sodium hydroxide *: Estimated input concentration **: Fully soluble, input material was not sufficient to maintain a slurry ^{‡}: Not detected | | | | | |

For pH 1.2 succinate experiments there was insufficient available material at the time of experiment to maintain a suspension.

**Table 1b: Thermodynamic solubility results using 0.5 M buffers**

| Test compound | Buffer | Initial pH | 24 h pH | Solubility (mM freebase) |
|---|---|---|---|---|
| Acetate Salt (Form A) | pH 1.2 | 4.06 → 1.16 | 3.73 → 1.24 | 243.85 |
| | pH 4.5 | 4.60 | 4.60 | 1.05 |
| Fumarate Salt (Form D) | pH 1.2 | 1.46 → 1.19 | 1.23 | 7.84 |
| | pH 4.5 | 4.55 | 4.50 | 1.64 |
| Succinate Salt (Form B) | pH 1.2 | 2.06 → 1.30 | 2.23 → 1.26 | 98.20 |
| | pH 4.5 | 4.58 | 4.54 | 1.71 |

### Conclusion

The test compounds exhibited remarkably different solubilities, especially at low pH. Specifically, the acetate and succinate salts showed high solubility at pH 1.2, indicating the potential for using these compounds in applications where a high solubility at low pH is desirable, such as the use in immediate release pharmaceutical formulations for gastric delivery.

### Example 2: Further solubility studies of AP1189 salts

### Solubility of AP1189 acetate and AP1189 succinate

### Materials and methods

3.4 g of AP1189 succinate and 2.9 g of AP1189 acetate were added to separate vials containing 10.0 mL of buffer solution pH 1.2 (one determination per salt). For the AP1189 acetate and AP1189 succinate solutions, the pH was measured to 3.9 and 2.2, respectively. As a result, the pH was adjusted to 1.2 with concentrated hydrochloric acid in both solutions. Both sample preparations were diluted 500 times with the sample diluent (acetonitrile:water 1:1 v/v).

The diluted samples preparations were analysed by HPLC within 5 hours from preparation and the content of AP1189 was determined from the area under the curve by comparing to standard solutions of AP1189 acetate and AP1189 succinate respectively.

Equilibrium solubilities were also assessed at pH 4.5 and pH 6.8 following the procedure as described in WHO Technical Report Series 1019, 2019 annex 4: Protocol to conduct equilibrium solubility experiments for the purpose of Biopharmaceutics Classification System-based classification of active pharmaceutical ingredients for biowaiver.

### Results

The sample materials in both vials were fully dissolved prior to dilution. The solubilities of the test compounds at pH 1.2 are shown in Table 2. The solubitilies of the test compounds at pH 4.5 and pH 6.8 are shown in Table 3 where all purities were found to be within 92 % and 95 %.

**Table 2. Determined concentration of AP1189 acetate and AP1189 succinate in pH 1.2 samples. The HPLC purities are also included in the table. The first purity results are obtained from the CoAs for the lots and the second purity results were measured in the solubility experiment.**

| Sample | pH 1.2 (time) | HPLC purity |
|---|---|---|
| AP1189 acetate | > 617 mM (5 h) | 99.3 % → 92.5 % |
| | > 223 mg/mL | |
| AP1189 succinate | > 593 mM (0.5 h) | 99.4 % → 91.9 % |
| | > 245 mg/mL | |

**Table 3. Summarized equilibrium solubilities (including standard deviations) for AP1189 acetate and AP1189 succinate at 37 °C in buffer pH solutions 4.5 and 6.8. The HPLC purities are also included in the table. The first purity results are obtained from the CoAs for the lots and the second purity results were measured in the solubility experiment.**

| Sample | pH 4.5 (time) | pH 6.8 (time) |
|---|---|---|
| AP1189 acetate | 3.2 mM ± 0.0 mM (22 h) | 2.4 mM ± 0.0 mM (22 h) |
| | 284 mg ± 3 mg/250 mL | 216 mg ± 2 mg/250 mL |
| | 99.3 % → 91.9 % | 99.3 % → 94.5 % |
| AP1189 succinate | 3.6 mM ± 0.0 mM (22 h) | 2.5 mM ± 0.1 mM (22 h) |
| | 370 mg ± 3 mg/250 mL | 261 mg ± 13 mg/250 mL |
| | 99.4 % → 92.2 % | 99.4 % → 93.7 % |

*Solubility of additional AP1189 salts*

| AP1189 salt form | Counterion trivial and/or systemic name | Solubility HCI/KCI Buffer 0.5 M pH 1.2 | Salt form crystallised from |
|---|---|---|---|
| Napadisylate (Form III, IV) | Naphthalene-1,5-disulfonic acid | <15 mM | 2-propanol:water 90:10 %v/v (Form III) or THF (Form IV) |
| Esylate (Form V) | Ethanesulfonic acid | <15 mM | methylethyl ketone |
| Edisylate (Form VII) | Ethane-1,2-disulfonic acid | <15 mM | methylethyl ketone |
| Nitrate (Form X) | Nitric acid | | THF |
| Cyclamate (Form XI, XII) | Cyclohexylsulfamic acid | <15 mM | THF (Form XI) or acetone (Form XII) |
| Besylate (Form XIV) | Benzenesulfonic acid | ≥ 15 to < 50 mM | 2-Propanol:water 80:20 %v/v |
| Oxalate (Form XV, XVI, XVII) | Oxalic acid | <15 mM | 2-Propanol:water 80:20 %v/v (Form XV) or acetone (Form XVI) or THF (Form XVII) |
| (+)-Camphor-10-sulfonic acid (Form XVIII) | (+)-Camphor-10-sulfonic acid | <15 mM | 2-Propanol:water 80:20 %v/v |
| Oxoglutarate (Form XIXI) | 2-oxoglutaric acid, ketoglutaric acid, 2-oxopentanedioic acid | ≥ 15 to < 50 mM | acetone |
| DL-Mandelic acid (Form XX) | Hydroxy(phenyl)ac etic acid | ≥ 50 mM | methylethyl ketone |
| Hippuric acid (Form XXII) | N-Benzoylglycine | ≥ 50 mM | methylethyl ketone |
| Formic acid (Form XXIII) | Formic acid | ≥ 15 to < 50 mM | acetone |
| L-Lactic acid (Form XXIV) | 2-Hydroxypropanoic acid | ≥ 50 mM | acetone |
| DL-Lactic acid (Form XXV) | 2-Hydroxypropanoic acid | ≥ 15 to < 50 mM | 2-propanol:water 80:20 %v/v |
| Glutaric acid (Form XXVI) | Pentanedioic acid | ≥ 15 to < 50 mM | acetone |
| Adipic acid (Form XXIX) | Hexanedioic acid | ≥ 15 to < 50 mM | 2-Propanol:water 80:20 %v/v |

### Example 3: Dissolution of AP1189 acetate and AP1189 succinate tablets

A dissolution experiment comparing the dissolution behaviour of the powder formulation based on AP1189 acetate with the dissolution behaviour for 200 mg tablet manufactured based on the acetate salt of AP1189 and succinate salt of AP1189.

### Materials and methods

Tablets: RDT2101-6-T3-C1, AP1189 acetate (200 mg AP1189 acetate, equivalent of 167 mg AP1189)
Tablets: RD2103-2-T1-C1, AP1189 succinate ( Equivalent of 200 mg AP1189 acetate, 167 mg AP1189)
Powder: AP1189 acetate (200 mg AP1189 acetate) + Syrspend^{®} SF Alka, suspended in 50 ml water.

The dissolution experiment was conducted in a USP 2 paddle equipment at 37 °C. The dissolution media consist of 500 ml 0.1 N HCl + 50 ml of water is added to the chamber after addition of the tablet. Rotation speed: 50 rpm. Sampling time points: 5, 10, 15, 30, 45, and 60 min.

### Results

The three products behaved nearly identical on the exposure to acidic conditions simulating stomach condition. They were all immediate release products. At 5 minutes, 65 - 80 % of the APIs were dissolved. At 10 minutes, all 3 products had reached their maximum dissolution at approximately 80 %. The lower than 100 % release was identified to be due to instability of the dissolution samples prior to analysis in the HPLC sequence.

### Conclusion

2 prototype tablets of AP1189 based on respectively the succinate or the acetate salt showed nearly identical dissolution properties when compared to the powder formulation.

### Example 4: Degradation of phenyl pyrrole aminoguanidine compounds in acidic conditions

Acidic conditions are likely to degrade the aminoguanidine derivatives disclosed in WO 2007/141343, including degradation by hydrolysis of the imine moiety.

Stability of AP1189 in Ora-Sweet and Ora-Sweet SF was tested. Approximately 3.75 mg of AP1189 was suspended in 5 ml Ora-Sweet or Ora-Sweet SF and the suspension was analysed by HPLC. No interference was observed from Ora-Sweet or Ora-Sweet SF to PA1189 peak. However a sharp degradation peak was observed at RRT 0.88 (>5.1% Total area) which indicates that AP1189 is unstable in Ora-sweet and Ora-Sweet SF.

### A short study was conducted to further determine the stability of AP1189 in Ora-Sweet SF.

A suspension was made at 20 mg/ml and 3.5 mg/ml concentrations (respective to the 400 mg and 15 mg formulations) and analysed by HPLC at initial (T=0) and T=1 hour interval. Results show that AP1189 degradation in Ora-Sweet SF within 1 hour, which may be corresponding to its pH (pH 4.25):

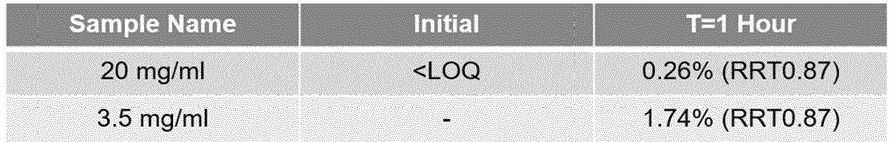

Further to the findings that AP1189 is unstable in Ora Sweet SF an alternative alkaline vehicle (pH >7.0) Syrspend SF Alka was used to suspend the AP1189. An experiment was conducted at lowest (15mg) and highest (400mg) dose strength to assess the dispersability and physical stability of the suspension. Chemical stability of the suspension was assessed by HPLC for 90 minutes at every 30 minutes interval. Results shows the API as stable in Syrspend SF Alka for 90 minutes (room temp.):

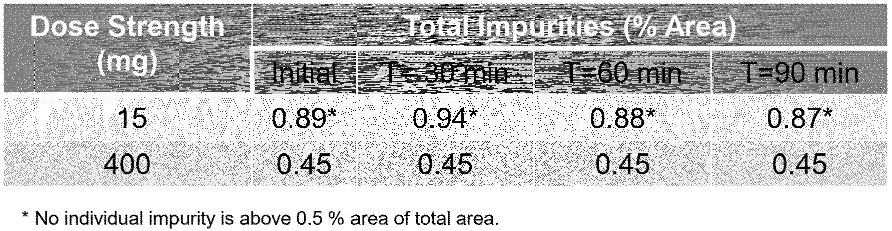

Forced degradation studies on AP1189 has also demonstrated degradation at low pH, were at least 20% degradation occurred within 24 hours. Rapid pH dependent degradation has also been demonstrated in Examples 2 and 3 herein.

Protection of AP1189 from degradation has thus been attempted by suspending AP1189 acetate in an alkaline medium (Syrspend^{®} SF Alka suspension) and from gastric acids by enteric coating tablets (cf. examples below).

### Example 5: Enteric coated tablet provides low absorption of AP1189 at single dose

Based on the finding that AP1189 is degraded in an acid environment (see example 4), attempts to protect AP1189 from gastric acids were made by the formulation of an enterically coated tablet. An enteric coating is a polymer barrier applied to oral medications that prevents its dissolution or disintegration in the gastric environment, thus protecting drugs from the acidity of the stomach. The drug will be released after the stomach (usually in the upper tract of the intestine).

### Materials and methods

AP1189 acetate powder for oral suspension was provided in individual vials, each vial containing 200 mg. AP1189 acetate powder was dispersed in a vehicle (Syrspend^{®} SF Alka) and administered as an oral suspension. The alkaline excipient was chosen to protect the API against degradation by acid.

100 mg enteric coated AP1189 tablets manufactured by wet granulation followed by compression and coating, were comprising the components as listed in Table 4:

**Table 4: Components in 100 mg enteric coated AP1189 tablets.**

| | **Component (trade name)** | **Percent Formula (%w/w)** | **Content per tablet (mg)** |
|---|---|---|---|
| **Intra-granular** | AP1189, acetate | 30.645 | 122.58 |
| | Dibasic calcium phosphate anhydrous (A-comprez powder) | 37.105 | 148.42 |
| | Microcrystalline cellulose (Avicel PH101) | 20.00 | 80.00 |
| | Crospovidone (Kollidon CL) | 5.00 | 20.00 |
| | Hydroxypropyl cellulose (Klucel EXF) | 5.00 | 20.00 |
| **Granulating fluid** | Sodium lauryl sulphate (Empicol LXN) | 1.00 | 4.00 |
| | Sterile water ** | N/A | N/A |
| **Extra-granular** | Magnesium stearate (Ligamed) | 1.25 | 5.00 |
| | **Core Tablet Total** | **100.00** | **400.00** |
| **Enteric coating** | Acryl-EZE II Yellow (493Z210006) | N/A | 48.00 |
| | Sterile water | N/A | N/A |
| | **Total** | **N/A** | **448.00** |

Treatment was administered as an oral suspension in fasting condition and as enteric coated tablets in fed or fasting condition according to the allocated sequence of treatments. AP1189 tablets were given with 240 mL of still mineral water at room temperature. Subjects were dosed whilst standing or semi-recumbent and were not allowed to lie flat for 4 h post-dose, except for study procedures or if clinically indicated.

The study was carried out on 8 healthy male volunteers, aged 22 to 38 years old and with BMI between 21.6 and 28.7 kg/m². Each subject received on 3 single occasions, separated by at least 7 days of wash-out, a single AP1189 oral dose of 200 mg. Each subject received in a randomized order, each of the following treatments:
- Treatment A: AP1189 oral suspension (alkaline) corresponding to 200 mg in fasting conditions,
- Treatment B: 2 enteric coated tablets dosed at 100 mg AP1189 per tablet corresponding to 200 mg AP1189 by oral route in fasting conditions,
- Treatment C: 2 enteric coated tablets dosed at 100 mg AP1189 per tablet corresponding to 200 mg AP1189 by oral route in fed conditions (high-fat breakfast).

On each treatment period, blood samples were collected for AP1189 (and its identified metabolites if any) determinations at the following time points: pre-dose, then 0.25, 0.50, 0.75, 1.00, 1.50, 2.00, 3.00, 4.00, 6.00, 8.00, 12.00, 16.00, 24.00, 36.00, 48.00, 72.00, 96.00 and 120.00 h post-dose (N = 57).

### Results

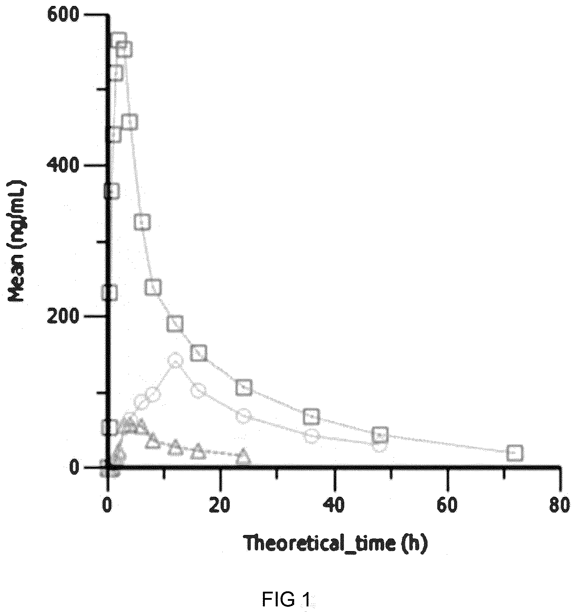

Figure 1 shows the mean AP1189 plasma concentration vs. time by treatment. Regarding PK results, 3 subjects out of 8 did not have measurable concentrations of AP1189 following dosing with the tablet under fasted condition whereas all subjects presented measurable levels after the suspension.

With the enteric coated tablet, mean Cₘₐₓ was decreased by 9.2 compared to the oral suspension (63.84 ng/mL vs. 589.40 ng/mL). The overall exposure as expressed by AUC₀₋ₜ was also decreased by a factor 5.6 (mean AUC₀₋ₜ of about 8401 ng.h/mL with suspension vs. 1501 ng.h/mL with tablet). AUC_{0-∞} could be determined only in 2 subjects with tablet. Relative bioavailability based on AUC₀₋ₜ median was 10% with high inter-individual variability (104%). Median tₘₐₓ was delayed with tablet (4 h vs. 2 h). Individual values were between 1.5 and 3 h post-dose with suspension and between 3 and 4 h post-dose with tablet. Mean value of t_{1/2} was about 19.8 h with suspension and 17.0 h with tablet (determined in only 4 subjects out of 8). Inter-individual variability was low with CV% below 21% with the 2 formulations. For Cₘₐₓ and AUC₀₋ₜ, inter-individual variability expressed as CV% was low with suspension (around 19%) whereas it was very high with tablet (between 116 and 142%). With suspension, mean Vd/F was about 667 L and CL/F was about 23 L/h with inter-individual variability around 20%.

In fasting condition, Cₘₐₓ was about 3 times lower than in fed condition (63.84 ng/mL vs. 194.95 ng/mL). The overall exposure as expressed by AUC₀₋ₜ was also lower (1501.22 ng.h/mL in fasting condition vs. 3550.08 ng.h/mL in fed condition). AUC_{0-∞} could be determined only in 2 subjects in fasting conditions. Relative bioavailability based on AUC₀₋ₜ median was 718% with high inter-individual variability (90%).

Median tₘₐₓ was delayed in fed condition (12 h *vs.* 4 h). Individual values were between 3 and 4 h post-dose in fasting condition and between 4 and 24 h post-dose in fed condition. Mean value of t_{1/2} was about 17.0 h with tablet in fasting condition (only 4 subjects out of 8) and 19.6 h with tablet in fed condition. Inter-individual variability was low with CV% below 22% regardless the conditions of administration. For Cₘₐₓ and AUC₀₋ₜ, inter-individual variability expressed as CV% was very high in fasting condition (between 116 and 142%) whereas it was moderate in fed condition (between 43 and 45%). In fed condition, mean Vd/F was about 1297 L and CL/F was about 50 L/h with inter-individual variability around 25%.

With enteric coated tablet in fed condition, mean Cₘₐₓ was decreased by 3 compared to oral suspension in fasting condition (194.95 ng/mL vs. 589.40 ng/mL). The overall exposure as expressed by AUC₀₋ₜ was also decreased by a factor 2.4 (mean AUC₀₋ₜ of about 8401 ng.h/mL with suspension in fasting condition vs. 3550 ng.h/mL with tablet in fed condition). AUC_{0-∞} was decreased by a factor 1.9 (mean AUC_{0-∞} of about 8812 ng.h/mL with suspension in fasting condition vs. 4522 ng.h/mL with tablet in fed condition). Relative bioavailability based on AUC₀₋ₜ median was 40% with high inter-individual variability (53%). Median tₘₐₓ was delayed with tablet in fed condition compared to suspension in fasting condition (12 h *vs.* 2 h). Individual values were between 1.5 and 3 h post-dose with suspension and between 4 and 24 h post-dose with tablet. Mean value of t_{1/2} was about 19.8 h with suspension and 19.6 h with tablet. Inter-individual variability was low with CV% below 22% with the 2 formulations. For Cₘₐₓ and AUC₀₋ₜ, inter-individual variability expressed as CV% was low with suspension (around 19%) whereas it was moderate with tablet in fed condition (between 26 and 45%).

### Conclusion

AP1189 acetate exposure (Cₘₐₓ and AUCs) highly decreased with the enteric coated tablet formulation compared to an oral suspension in fasting condition and tₘₐₓ was delayed (4 h vs. 2 h). Based on AUC0-t relative bioavailability was 10% between suspension and enteric coated tablet in fasting condition. In fasting condition, formal statistics showed that bioavailability was statistically and significantly reduced with tablet compared to oral suspension. Food intake increased significantly AP1189 exposure after administration of the tablet formulation with a factor 2.4 for Cₘₐₓ and 3.9 for AUC₀₋ₜ. Furthermore tₘₐₓ was delayed (12 h *vs.* 4 h). Administration of single dose of AP1189 200 mg as suspension, tablet in fasting condition or tablet in fed condition in healthy male subjects was very well tolerated.

Data from the administration of the enteric coated tablet showed lower exposure with higher variability compared to data from the suspension, and a high and unpredicted degree of bioavailability both within and between subjects.

These findings demonstrate that formulating AP1189 in an enterically coated tablet formulation result in suboptimal absorption and thus low bioavailability of the API.

### Example 6: Enteric coated tablet provides low absorption of API at repeated dosages

### Materials and methods

100 mg enteric coated AP1189 tablets were prepared as described in Example 5, and placebo tablets were manufactured in a similar manner comprising the components listed in Table 5:

**Table 5: Components in placebo tablets to 100 mg enteric coated AP1189 tablets.**

| | **Component (Trade name)** | **Percent Formula (%w/w)** | **Content per tablet (mg)** |
|---|---|---|---|
| **Intra-granular** | Dibasic calcium phosphate Anhydrous (A-comprez powder) | 57.40 | 264.04 |
| | Microcrystalline cellulose (Avicel PH101) | 30.35 | 139.61 |
| | Crospovidone (Kollidon CL) | 5.00 | 23.00 |
| | Hydroxy propyl cellulose (Klucel EXF) | 5.00 | 23.00 |
| **Granulating fluid** | Sodium lauryl sulphate (Empicol LXN) | 1.00 | 4.60 |
| | Sterile water * | N/A | N/A |
| **Extra-granular** | Magnesium stearate (Ligamed) | 1.25 | 5.75 |
| | **Core Tablet Total** | **100.00** | **460.00** |
| **Enteric coating**** | Acryl-EZE II Yellow (493Z210006) | | 55.20 |
| | Sterile water * | | N/A |
| | **Total** | | **515.20** |

The study was performed on 12 subjects aged 20 to 39 years old with BMI between 19.6 and 27.4 kg/m². Each subject received on Day -8 a single 400 mg moxifloxacin oral dose, followed by a 14-day repeated q.d. AP1189 acetate (200 mg, two tablets) or placebo as enteric coated tablet administration period. Blood samples were collected at the following time points:
- For moxifloxacin determination on Day -8 at pre-dose, then 1.0, 2.0, 3.0, 4.0 and 5.0 h post-dose (N = 6),
- On Day -1, to perfectly mimic Day 14 conditions at pre-dose, then, 0.5, 1.0, 1.5, 2.0, 2.5, 3.0, 4.0, 5.0, 6.0, 8.0, 10.0, 12.0, and 16.0 h post-dose (N = 14).
- For AP1189 (and its identified metabolites, if any) determination:
   - On Day 1: pre-dose, then, 0.5, 1.0, 1.5, 2.0, 2.5, 3.0, 4.0, 5.0, 6.0, 8.0, 10.0, 12.0, and 16.0 h post-dose,
   - From Day 2 to Day 6: pre-dose,
   - On Day 7: pre-dose then 1.0, 2.0, 3.0, 4.0, 6.0, 8.0 and 12.0 h post-dose,
   - From Day 8 to Day 13: pre-dose,
   - On Day 14: pre-dose, then, 0.5, 1.0, 1.5, 2.0, 2.5, 3.0, 4.0, 5.0, 6.0, 8.0, 10.0, 12.0, and 16.0 h post-dose,
   - On Day 15: 24.0 and 36.0 h post-Day 14 administration,
   - On Day 16: 48.0 h post-Day 14 administration,
   - On Days 17, 18 and 19: 72.0, 96.0 and 120.0 h post-Day 14 administration (N = 53).

### Results

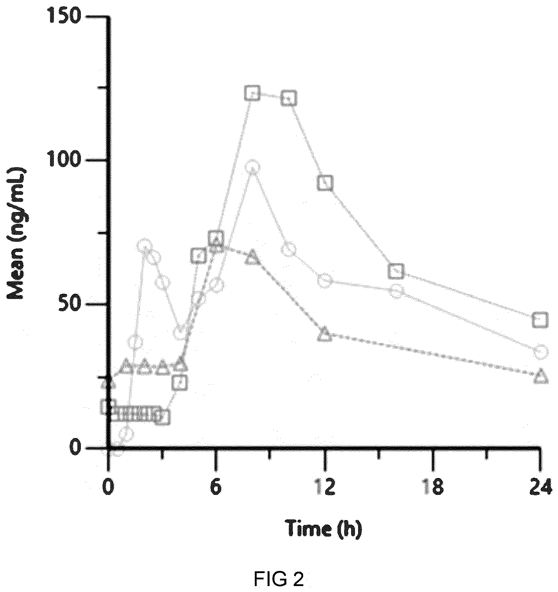

The meant AP1189 plasma concentrations are shown in Figure 2. For all the subjects, on Day 1, pre-dose sample did not contain quantifiable AP1189. No pre-dose samples were missing. On Day 1, 3 subjects had no measurable concentrations after administration. For other subjects, first quantifiable concentration was observed between 1 and 12 h. For these subjects concentrations were measurable up to 24 h post-dose (time of the next administration). On Day 7, 1 subject had no measurable concentrations after administration. For other subjects, first quantifiable concentration was observed between 0 and 12 h. Two (2) subjects had measurable concentration only at pre-dose and 12 h post-dose. In 6 subjects, concentrations were measurable up to 24 h post-dose (time of the next administration). On Day 14, first quantifiable concentration was observed between 0 and 10 h. Concentrations were measurable up to 16 h in 1 subject, 24 h in 3 subjects, 36 h in 2 subjects, 48 h in 1 subject and 72 h in the last subject.

### Conclusion

Following administration of 200 mg AP1189 acetate enteric tablet formulation, PK was highly unpredictable. PK of moxifloxacin was consistent with those described in the literature. Administration of multiple doses of AP1189 acetate 200 mg as tablet in fed condition in healthy male subjects was well tolerated.

### Example 7: Immediate release tablets of A1189 acetate and AP1189 succinate Materials and methods

Immediate release tablets of AP1190 acetate and AP1189 succinate were manufactured as exemplified for 200 mg tablets. Tablets with another dose can be produced in essentially the same manner by adjusting the amount of granulate and excipients in the compression step.

Granulation was performed in the same manner in a 1 L high shear mixer and dried in a drying cabinet at 40 °C.

AP1189 salt was mixed with filler (Microcrystalline cellulose), binder (Maltodextrin), polysorbate 80, and sodium starch glycolate and wetted with purified water until granulation was obtained.

**Table 6: Granulation batch composition with AP1189 salts.**

| Component | AP1189 acetate | AP1189 succinate |
|---|---|---|
| | Batch: RD2101-6 | Batch: RD2103-2 |
| | g (%) | g (%) |
| AP1189 acetate | 150 (75) | - |
| AP1189 Succinate | - | 151.06 (75.53) |
| Microcrystalline cellulose | 29.5 (14.75) | 28.44 (14.22) |
| Maltodextrin | 20 (10) | 20 (10) |
| Sodium starch glycolate | 0.5 (0.25) | 0.5 (0.25) |
| Polysorbate 80 | 0.2 (0.10) | 0.2 (0.10) |
| Purified water | 78.40 (N/A) | 80.00 (N/A) |
| Total | 200 (100) | 200 (100) |

### Compression of AP1189 tablets

The AP1189 granulates were mixed with fillers silicified microcrystalline cellulose and mannitol in a Turbula mixer. Magnesium stearate was added and mixing continued. All compression was done on the Diaf single punch press using 15.5 x 7.1 oval tooling.

**Table 7: Batch composition and in-process data from compression**

| Salt | AP1189 acetate | AP1189 succinate |
|---|---|---|
| Batch no. | RD2101-6-T3 | RD2103-2-T1 |
| Strength (declared as AP1189 acetate) | 200 mg | 200 mg |
| AP1189 granulate | 174.20 g / 67.00% | 156.05 g / 60.00% |
| Silicified Microcrystalline Cellulose | 62.40 g 24.00 % | 76.05 g / 29.25 % |
| Mannitol | 20.80 g / 8.00 % | 25.40 g / 9.75 % |
| Magnesium stearate | 2.60 g / 1.0% | 2.61 g / 1.0% |
| Batch size | 260 g/650 tablets | 260 g/506 tablets |
| Compression setting | 5 ½ | 6 2/3 |
| Compression speed | 36 tablets/min | 41 tablets/min |
| Tablet weight / RSD | 405.28 mg / 0.20% | 512.90 mg / 0.25% |
| Tablet thickness | 5.48 mm | 6.48 mm |
| Hardness | 84.3 N | 99.0 N |

### Film coating

Tablets were film coated with a white, standard Hypromellose film containing Titan Dioxide (Table 8). Film was dosed to achieve a weight gain of approx. 4%, which secure a uniform colour.

**Table 8: Film composition**

| | |
|---|---|
| Opadry 03F180011 WHITE | 30 g |
| Purified water | 270 g |
| Total | 300 g |

Table 9 gives the process parameters measured over the film coating processes and actual weight gains. All tablets appeared nice and smooth with uniform colour.

**Table 9: Film coating process parameters.**

| Salt | AP1189 acetate | AP1189 succinate |
|---|---|---|
| Batch no. | RD2101-6-T3-C1 | RD2103-2-T1-C1 |
| Strength (AP1189 as base) | 166.7 mg | 166.7 mg |
| Nominal strength (as acetate) | 200 mg | 200 mg |
| Inlet air temperature (°C) | 55.0 | 55.3 |
| Outlet air temperature (°C) | 44.7 | 42.4 |
| Coating solution spray rate (g/min) | 3.4 | 3.4 |
| Batch size (g core tablets) | 200 g | 220 g |
| Amount of coating solution (g) | 103.0 | 105.9 |
| Coated tablet weight (mg) | 418.29 | 537.88 |
| Weight gain (%) | 3.5 | 4.1 |
| Disintegration time | < 1 min | 1 min 20 sec |

### Analytical results

The batches were tested for assay, impurities, content uniformity, dissolution and disintegration. Results are given in Table 10.

**Table 10: Analytical results from AP1189 immediate release tablets**

| Salt | AP1189 acetate | AP1189 succinate |
|---|---|---|
| Batch no | RD2101-6-T3-C1 | RD2103-2-T1-C1 |
| Strength (AP1189 as base) | 166.7 mg | 166.7 mg |
| Appearance | White oval tablet | White oval tablet |
| Assay | 163.82 mg | 165.75 mg |
| | 98.4% | 99.45% |
| CoU (n=10) | 170.7 mg | 162.1 mg |
| Rel. std.dev %: | 4.04% | 1.62% |
| AV value(<15): | 10.8 | 5.0 |
| Impurities (Area %): | Largest: 0.41% | Largest: 2.42%* |
| | Total 1.16% | Total 5.15%* |
| Dissolution: | | |
| 5 min: | 68.6% | 73.3% |
| 10 min: | 79.9% | 82.2% |
| 15 min: | 81.9% | 83.8% |
| Disintegration: | < 1 min | 1 min 20 s |

| | | |
|---|---|---|
| * Purity of batch of AP1189 succinate used was comparable | | |

Dissolution results tend to stabilize around 85%. This may be due to instability of the AP1189 in acid environment during HPLC analysis.

Similarly, 50 mg AP1189 tablets were manufactured (cf. Tables 11-12 below):

**Table 11: Granulation batch composition with AP1189 salts.**

| Component | AP1189 acetate | AP1189 succinate |
|---|---|---|
| | | |
| | g (%) | g (%) |
| AP1189 acetate | 150 (75) | - |
| AP1189 Succinate | | 153.58 (76.79) |
| Microcrystalline cellulose | 29.3 (14.65) | 25.72 (12.86) |
| Maltodextrin | 20 (10) | 20 (10) |
| Sodium starch glycolate | 0.5 (0.25) | 0.5 (0.25) |
| Polysorbate 80 | 0.2 (0.10) | 0.2 (0.10) |
| Purified water | 79.80 (N/A) | 79.80 (N/A) |
| Total | 200 (100) | 200 (100) |

**Table 12: Batch composition for compression**

| Salt | AP1189 acetate | AP1189 succinate |
|---|---|---|
| Strength (declared as AP1189) | 50 mg | 50 mg |
| AP1189 granulate | 105.35 g / 21.07% | 113.64 g / 22.73% |
| Silicified Microcrystalline Cellulose | 292.25 g / 58.45 % | 286.02 g / 57.20 % |
| Mannitol | 97.40 g / 19.48 % | 94.34 g / 19.07 % |
| Magnesium stearate | 5.00 g / 1.0% | 5.00 g / 1.0% |
| Batch size | 500 g | 500 g |

### Conclusion:

A process for manufacturing immediate release tablets comprising AP1189 acetate and

AP1189 succinate respectively was successfully demonstrated.

### Example 8: Bioavailability of immediate release formulation of AP1189 acetate and succinate

The pharmacokinetic profile of an immediate release formulation of AP1189 acetate and succinate was assessed as detailed below.

### Materials and methods

Test items
- AP1189 acetate (200 mg) for suspension in SyrSpend SF Alka^{®} in water
- AP1189 acetate (AP1189 tablets, 200 mg, batch RD2101-6-T3-C1)
- AP1189 succinate (SP1189 tablets, 200 mg, batch RD2103-2-T1-C1)

### Dose formulation preparation

The Syrspend SF ALKA formulation (suspension) was prepared for each individual dose by mixing 200 mg AP1189 acetate with 3.15 gram Syrspend SF ALKA Unflavored (Fagron item no 33736-104). 50 mL water was added at room temperature and the suspension was shaken vigorously by hand. The ready formulation was administered within 90 minutes after preparation. All preparations were prepared in glass containers.

### Animals

The study was performed in 10 female Göttingen SPF minipigs (Ellegaard Göttingen Minipigs A/S, Denmark). Initially, 9 animals were allocated to the study. One additional animal was allocated from the internal stock on Day 9 of the study. The animals were 3 to 4 months old and with a body weight of 7.5 to 9 kg at arrival. A pre-treatment period of 15 days (including an acclimatisation period of 5 days) was allowed.

### Housing, diet

The study took place in filtered air at a temperature of 21°C ±3°C. The room was illuminated to give a cycle of 12 hours light and 12 hours darkness.

An SDS minipig diet (SMP (E) SQC) from Special Diets Services, Witham Essex, CM8 3AD, U.K., was offered twice daily in an amount of approximately 125 g per animal per meal. In order to allow a reasonable growth of the animals, the amount of diet was slightly adjusted. The animals had ad libitum access to domestic quality drinking water.

### Animal randomization and allocation

On the day of arrival, the animals were given a final number, using a randomisation scheme.

### Treatment

The groups, dose levels and animal numbers were as follows in Table 13 below.

**Table 13: Treatment schedule**

| Animal No | Group | No of days treated | Dose*, AP1189 equivalents mg/animal (dosing form) |
|---|---|---|---|
| 1 | 1 | 5 | 200 (Suspension, AP1189 acetate) |
| 2 | 1 | 5 | 200 (Suspension, AP1189 acetate) |
| 3 | 1 | 5 | 200 (Suspension, AP1189 acetate) |
| 4 | 2 | 5 | 200 (1 tablet AP1189) |
| 5 | 2 | 5 | 200 (1 tablet AP1189) |
| 6 | 2 | 5 | 200 (1 tablet AP1189) |
| 7 | 3 | 5 | 200 (1 tablet SP1189) |
| 8 | 3 | 2# | 200 (1 tablet SP1189) |
| 9 | 3 | 5 | 200 (1 tablet SP1189) |
| 10 | 4 | 2# | 200 (1 tablet SP1189) |

| | | | |
|---|---|---|---|
| *- Nominal dose (mg) (equivalent of mg AP1189 acetate) # - Animal No 8 was terminated for ethical reasons on Day 2 of the study and replaced by animal No 10 to ensure a full PK profile but was also terminated prematurely. | | | |

Treatment was for 5 days. The first day of treatment was designated Day 1.

For Group 1 animals, the daily dose was given orally for 5 days by oral gavage in a fixed volume of 50 mL/animal. Administration of the test formulation in suspension was followed by ca 10 mL tap water.

For Group 2 and Group 3 animals, the daily dose was given orally in tablet formulation. Administration of one tablet was followed by 60 mL tap water (10 mL in a syringe to assure tablet passage, and 50 mL by oral gavage). For 5 days, animals received 1 tablet/day.

### Results

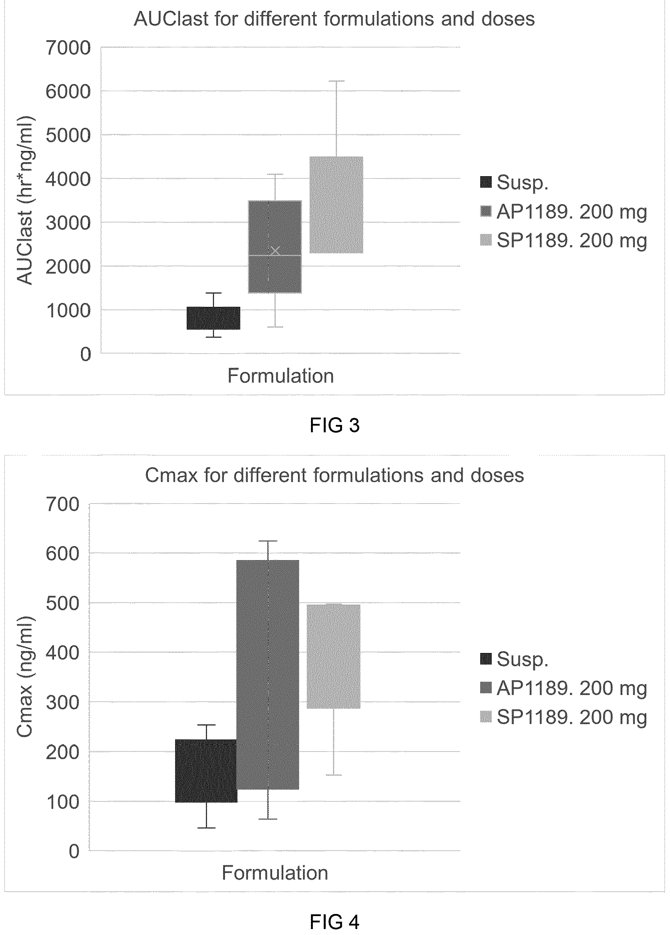

Figure 3 shows a comparison of the effect of the three different formulations on the AUC. Figure 4 shows a comparison of the effect of the three different formulations on Cₘₐₓ.

### Conclusion

Exposure to AP1189 was confirmed for all dosed animals regardless of formulation administered. The exposure (Cₘₐₓ and AUC₀₋₂₄) for the AP1189 suspension appeared to be lower than for both AP1189 (AP1189 acetate) and SP1189 (AP1189 succinate) tablets. For all formulations, the Tₘₐₓ was found at either 1 or 2 hours post dosing and elimination half-life was estimated to about 4-5 hr. These findings demonstrate that an immediate release solid oral formulation of AP1189 acetate or AP1189 succinate targeting the gastric compartment facilitate high absorption of AP1189.

### Example 9: Comparative bioavailability study in humans

A single center, open label, 3-part pharmacokinetic (PK) study was conducted. Twelve healthy male Caucasian subjects were randomized. Each subject received on three separate occasions, in a randomized order, each of the following single oral dose:
- Treatment A: 100 mg AP1189 powder for suspension corresponding to 83 mg AP1189 in fasting conditions,
- Treatment B: Two 50 mg AP1189 tablets (AP1189 acetate) corresponding to 100 mg AP1189 in fasting conditions,
- Treatment C: Two 50 mg SP1189 tablets (AP1189 succinate) corresponding to 100 mg AP1189 in fasting conditions.

Blood samples were collected from all subjects for PK purpose for AP1189 determinations, on each study period, at the following time points: pre-dose, then 0.25, 0.5, 0.75, 1.0, 1.5, 2.0, 3.0, 4.0, 6.0, 8.0, 12.0, 16.0, 24.0, 36.0, 48.0, 72.0, 96.0 and 120.0 h post-dose.

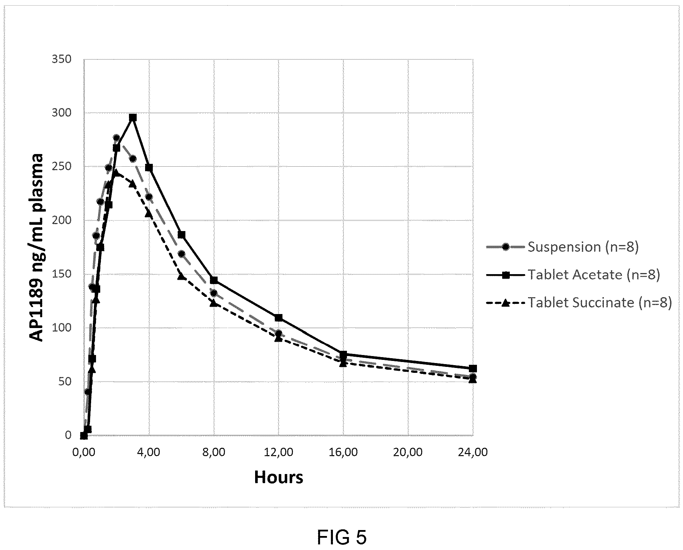

After 2 dosing rounds (n=8 for each dosing form), plasma samples were analyzed for AP1189. Derivation of PK parameters were carried out using Phoenix WinNonlin^{®} (Version Phoenix 8.1 - Certara - Princeton - USA). A non-compartmental analysis (NCA) approach was chosen. The results are shown in Tables 14 and 15 herein below, and presented in figure 5.

Three subjects in the Treatment A group (receiving AP1189 acetate in suspension) experienced nausea, and one of the subjects vomited, after administration. These effects were not observed in the Treatment B and C groups.

The following PK parameters of AP1189 were derived for each subject receiving AP1189 on Day 1 of each treatment period:
- **t_{lag}**: The time before start of absorption was also obtained directly from the concentration-time data; it was the time point immediately prior to the first quantifiable plasma concentration.
- **Cₘₐₓ**: The observed maximum concentration measured in plasma was obtained directly from the concentration-time data.
- **Cₘₐₓ/D**: Cₘₐₓ divided by the dose
- **tₘₐₓ**: The time at which Cₘₐₓ was apparent, identified by inspection of the plasma drug concentration *versus* (*vs.)* time data by Phoenix^{®} WinNonlin^{®}.
- **AUC₀₋ₜ**: The area under the concentration-time curve from time zero (pre-dose) to the time of last quantifiable concentration was calculated using a linear trapezoidal method (at least 3 concentrations above LOQ were required).
- **AUC₀₋ₜ/D**: AUC₀₋ₜ divided by the dose
- **kₑ**: The terminal plasma elimination rate-constant was estimated from log-linear regression analysis of the terminal phase of the plasma concentration-time profile. The number of points included in the terminal phase was determined by visual inspection of the semi-log plots of the plasma concentration-time profiles. A minimum of 3 data points, including the last measured data point and excluding Cₘₐₓ, should be available for the regression. The coefficient of determination for the goodness of the fit of the regression line through the data points (r²) had to be 0.90 or higher, for the value to be considered reliable. No determination of derived parameters (t_{1/2}, AUC extrapolation, Vd/F or CL/F) were performed with unreliable elimination rate constants. (hence, considered as not calculable - NC).
- **t_{½}**: The apparent terminal elimination half-life was calculated as In2/kₑ, where kₑ was the elimination rate constant as defined above.
- **AUC_{0-∞}**: The AUC from time 0 to infinity was calculated as AUC_{0-∞}= AUC₀₋ₜ + AUC_{t-∞}, where AUC₀₋ₜ was the area under the concentration-time curve from time zero (pre dose) to the time of last quantifiable concentration calculated using a linear trapezoidal method and AUC_{t-∞} = Ct/kₑ, where Ct was the measured concentration at time of the last quantifiable concentration t. The AUC was only calculated if there were at least 3 concentrations above LOQ. The extrapolated part of AUC_{0-∞} had to be < 20% for the value be considered as reliable.
- **AUC_{0-∞}/D**: AUC_{0-∞} divided by the dose
- **CL/F**: The apparent volume of the central compartment cleared of drug per unit time was estimated using the formula: CL/F = Dose / AUC_{0-∞}
- **Vd/F**: The apparent volume of distribution based on the terminal elimination phase. The estimate did not account for the bioavailability (F, as a fraction of 1) and was therefore nominally divided by this value when drug was given via extravascular routes. Vd/F=CL/F / kₑ
- **Fᵣₑₗ**: The relative bioavailability of the tablet formulation compared to the solution was obtained by the ratio of individual dose normalized AUC_{0-∞} values obtained after administration of each formulation (solution being the reference formulation).

**Table 14: Summary of main PK parameters by treatment:**

| **Treatment** | | **t_{lag}* (h)** | **tₘₐₓ* (h)** | **Cₘₐₓ (ng/mL)** | **AUC₀₋ₜ (h*ng/mL)** | **AUC_{0-∞} (h*ng/mL)** |
|---|---|---|---|---|---|---|
| **A** | **N** | 8 | 8 | 8 | 8 | 8 |
| | **Mean** | 0.00 | 2.00 | 257.27 | 3192.50 | 3705.07 |
| | **SD** | 0.09 | 1.02 | 41.319 | 590.14 | 675.55 |
| | **CV%** | 0.00-0.25 | 1.00-4.00 | 16.1 | 18.5 | 18.2 |
| | **GM** | | 1.86 | 254.48 | 3143.82 | 3650.17 |
| **B** | **N** | 8 | 8 | 8 | 8 | 8 |
| | **Mean** | 0.25 | 3.00 | 314.52 | 4500.78 | 4905.81 |
| | **SD** | 0.13 | 0.62 | 97.709 | 937.86 | 894.26 |
| | **CV%** | 0.00-0.25 | 1.50-3.00 | 31.1 | 20.8 | 18.2 |
| | **GM** | | 2.49 | 302.70 | 4423.40 | 4840.61 |
| **C** | **N** | 8 | 8 | 8 | 8 | 8 |
| | **Mean** | 0.25 | 2.00 | 260.28 | 3605.80 | 4084.24 |
| | **SD** | 0.12 | 0.88 | 48.448 | 827.97 | 825.53 |
| | **CV%** | 0.00-0.25 | 1.50-4.00 | 18.6 | 23.0 | 20.2 |
| | **GM** | | 2.25 | 255.93 | 3526.23 | 4013.18 |

| **Treatment** | | **t_{1/2} (h)** | **Vd/F (L)** | **CL/F (L/h)** |
|---|---|---|---|---|
| **A** | **N** | 8 | 8 | 8 |
| | **Mean** | 21.46 | 696.70 | 23.09 |
| | **SD** | 5.46 | 125.70 | 4.31 |
| | **CV%** | 25.5 | 18.0 | 18.7 |
| | **GM** | 20.89 | 685.44 | 22.74 |
| **B** | **N** | 8 | 8 | 8 |
| | **Mean** | 20.58 | 629.30 | 20.92 |
| | **SD** | 2.65 | 170.45 | 3.38 |
| | **CV%** | 12.9 | 27.1 | 16.2 |
| | **GM** | 20.43 | 609.00 | 20.66 |
| **C** | **N** | 8 | 8 | 8 |
| | **Mean** | 19.64 | 703.86 | 25.35 |
| | **SD** | 3.08 | 95.92 | 4.93 |
| | **CV%** | 15.7 | 13.6 | 19.5 |
| | **GM** | 19.42 | 698.27 | 24.92 |

| | | | | |
|---|---|---|---|---|
| *Median and Min-Max instead of mean and CV%, respectively. | | | | |

**Table 15: Summary of PK parameters dose-normalized and Frel by treatment:**

| **Treatment** | | Cₘₐₓ/D **(ng/mL/mg)** | **AUC₀₋ₜ/D (h*ng/mL/mg)** | **AUC_{0-∞}/D (h*ng/mL/mg)** | **Frel** |
|---|---|---|---|---|---|
| **A** | **N** | 8 | 8 | 8 | - |
| | **Mean** | 3.10 | 38.46 | 44.64 | - |
| | **SD** | 0.50 | 7.11 | 8.14 | - |
| | **CV%** | 16.1 | 18.5 | 18.2 | - |
| | **GM** | 3.07 | 37.88 | 43.98 | - |
| **B** | **N** | 8 | 8 | 8 | 4 |
| | **Mean** | 3.15 | 45.01 | 49.06 | 1.01 |
| | **SD** | 0.98 | 9.38 | 8.94 | 0.21 |
| | **CV%** | 31.1 | 20.8 | 18.2 | 20.8 |
| | **GM** | 3.03 | 44.23 | 48.41 | 1.00 |
| **C** | **N** | 8 | 8 | 8 | 4 |
| | **Mean** | 2.60 | 36.06 | 40.84 | 0.94 |
| | **SD** | 0.48 | 8.28 | 8.26 | 0.08 |
| | **CV%** | 18.6 | 23.0 | 20.2 | 8.4 |
| | **GM** | 2.56 | 35.26 | 40.13 | 0.93 |

## Claims

1. An immediate release solid oral dosage form comprising a pharmaceutically acceptable salt of AP1189 ((*E*)-*N*-[1-(2-nitrophenyl)-1-*H*-pyrrole-2-yl-allylideneamino]-guanidine) of formula I: 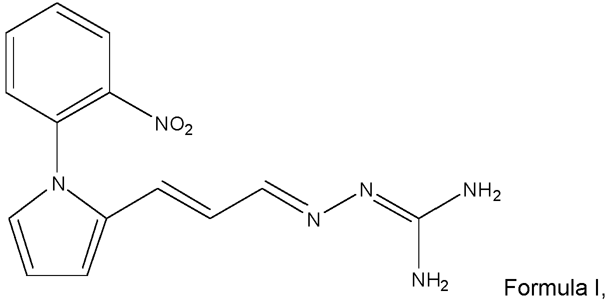
including tautomeric and stereoisomeric forms thereof,
wherein said pharmaceutically acceptable salt is selected from the group consisting of:
(*E*)-*N*-[1-(2-nitrophenyl)-1-*H*-pyrrole-2-yl-allylideneamino]-guanidinium acetate, including tautomeric and stereoisomeric forms thereof; and
(*E*)-*N*-[1-(2-nitrophenyl)-1-*H*-pyrrole-2-yl-allylideneamino]-guanidinium succinate, including tautomeric and stereoisomeric forms thereof;
and further comprising at least one pharmaceutically acceptable excipient selected from the group consisting of a binder, a disintegrant and a wetting agent.

2. The immediate release solid oral dosage form according to claim 1, wherein the pharmaceutically acceptable salt of AP1189 is (*E*)-*N*-[1-(2-nitrophenyl)-1-*H-*pyrrole-2-yl-allylideneamino]-guanidinium acetate, including tautomeric and stereoisomeric forms thereof (AP1189 acetate).

3. The immediate release solid oral dosage form according to claim 1, wherein the pharmaceutically acceptable salt of AP1189 is (*E*)-*N*-[1-(2-nitrophenyl)-1-*H-*pyrrole-2-yl-allylideneamino]-guanidinium succinate, including tautomeric and stereoisomeric forms thereof (AP1189 succinate).

4. The immediate release solid oral dosage form according to any one of claims 1-3, wherein said solid oral dosage form is an immediate release tablet.

5. The immediate release solid oral dosage form according to any one of claims 1-4, wherein not less than 80% of the salt dissolves into aqueous solution in 15 minutes at a pH of 1 to 3, using a USP 2 paddle equipment at 37 °C at a rotation speed of 50 rpm, wherein the dissolution media consist of 500 ml 0.1 N HCl + 50 ml of water, added to the dissolution chamber after addition of the tablet.

6. The immediate release solid oral dosage form according to any one of claims 1-5, wherein not less than 80% of the salt dissolves into aqueous solution in 15 minutes at a pH of 1.2, using a USP 2 paddle equipment at 37 °C at a rotation speed of 50 rpm, wherein the dissolution media consist of 500 ml 0.1 N HCl + 50 ml of water, added to the dissolution chamber after addition of the tablet.

7. The immediate release solid oral dosage form according to any one of claims 1-6, wherein said solid oral dosage form comprises said pharmaceutically acceptable salt of AP1189 in an amount of 25 mg to 250 mg (calculated as the free base); and wherein not less than 80% of the salt dissolves into aqueous solution in 15 minutes at a pH of 1 to 3, using a USP 2 paddle equipment at 37 °C at a rotation speed of 50 rpm, wherein the dissolution media consist of 500 ml 0.1 N HCl + 50 ml of water, added to the dissolution chamber after addition of the tablet.

8. The immediate release solid oral dosage form according to any one of claims 1-7, wherein Cₘₐₓ of the solid oral dosage form is not less than 180 ng/mL, such as not less than 200 ng/mL, such as not less than 225 ng/mL, such as not less than 250 ng/mL, such as not less than 275 ng/mL, such as not less than 300 ng/mL, such as not less than 350 ng/mL, such as not less than 400 ng/mL, such as not less than 450 ng/mL, such as not less than 500 ng/mL, such as not less than 600 ng/mL, such as not less than 700 ng/mL, such as not less than 800 ng/mL, such as not less than 900 ng/mL, such as not less than 1000 ng/mL, such as not less than 1200 ng/mL, such as not less than 1400 ng/mL, such as not less than 1600 ng/mL, such as not less than 1800 ng/mL, such as not less than 2000 ng/mL, such as not less than 2200 ng/mL, such as not less than 2400 ng/mL, such as not less than 2600 ng/mL, such as not less than 2800 ng/mL, such as not less than 3000 ng/mL, such as not less than 3200 ng/mL, such as not less than 3400 ng/mL, such as not less than 3600 ng/mL after administration of a single dose of said pharmaceutically acceptable salt of AP1189.

9. The immediate release solid oral dosage form according to any one of claims 1-8, wherein the pharmaceutically acceptable salt of AP1189 is present at a dosage of 25 mg to 650 mg AP1189 per dosage form, such as 25 mg, such as 50 mg, such as 100 mg, such as 150 mg, such as 200 mg, such as 250 mg, such as 300 mg, such as 350 mg, such as 400 mg, such as 450 mg, such as 500 mg, such as 550 mg, such as 600 mg, such as 650 mg of the pharmaceutically acceptable salt of AP1189 calculated as the free base.

10. The immediate release solid oral dosage form according to any one of claims 1-9, wherein the pharmaceutically acceptable salt of AP1189 is a polymorphic form of (*E*)-*N*-[1-(2-nitrophenyl)-1-*H*-pyrrole-2-yl-allylideneamino]-guanidinium acetate, including tautomeric and stereoisomeric forms thereof (AP1189 acetate), or a polymorphic form of (*E*)-*N*-[1-(2-nitrophenyl)-1-*H*-pyrrole-2-yl-allylideneamino]-guanidinium succinate, including tautomeric and stereoisomeric forms thereof (AP1189 succinate).

11. An immediate release solid oral dosage form according to any one of the preceding claims for use in treating or preventing a disease selected from a kidney disease, an arthritic disease such as rheumatoid arthritis, an inflammatory viral disease or disorder such as a viral respiratory disease or disorder, a cardiovascular disease, atherosclerosis, and a systemic inflammatory disorder such as an autoimmune disorder.

12. The immediate release solid oral dosage form according to any one of claims 1-10, or the use according to claim 11, wherein said pharmaceutically acceptable salt of AP1189 is administered in an amount of 1 mg to 1000 mg, such as 1 to 5 mg, 5 to 10 mg, 10 to 15 mg, 15 to 20 mg, 20 to 25 mg, 25 to 50 mg, 50 to 75 mg, 75 to 100 mg, 100 to 125 mg, 125 to 150 mg, 150 to 175 mg, 175 to 200 mg, 200 to 250 mg, 250 to 300 mg, 300 to 350 mg, 350 to 400 mg, 400 to 450 mg, 450 to 500 mg, 500 to 600 mg, 600 to 700 mg, 700 to 800 mg, 800 to 900 mg, or 900 to 1000 mg of the pharmaceutically acceptable salt of AP1189, per dosage calculated as the free base.

13. The immediate release solid oral dosage form according to any one of claims 1-10, or the use according to claims 11-12, wherein said pharmaceutically acceptable salt of AP1189 is administered once a day (QD), twice a day (BID) or three times a day (TID).

14. The immediate release solid oral dosage form according to any one of claims 1-10, or the use according to claims 11-12, wherein said pharmaceutically acceptable salt of AP1189 is administered at a once daily dosage of 1 to 1000 mg.

## Patentansprüche

1. Feste orale Dosierungsform mit sofortiger Freisetzung, umfassend ein pharmazeutisch verträgliches Salz von AP1189 ((*E*)-*N*-[1-(2-Nitrophenyl)-1-*H*-pyrrol-2-yl-allylidenamino]-guanidin) der Formel I: 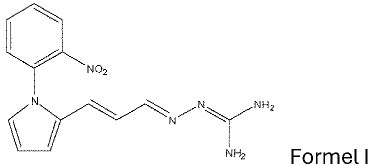 einschließlich tautomerer und stereoisomerer Formen davon, wobei das pharmazeutisch verträgliche Salz ausgewählt ist aus der Gruppe, bestehend aus:
(*E*)-*N*-[1-(2-Nitrophenyl)-1-*H*-pyrrol-2-yl-allylidenamino]-guanidiniumacetat, einschließlich tautomerer und stereoisomerer Formen davon; und
(*E*)-*N*-[1-(2-Nitrophenyl)-1-*H*-pyrrol-2-yl-allylidenamino]-guanidiniumsuccinat, einschließlich tautomerer und stereoisomerer Formen davon;
und ferner umfassend mindestens einen pharmazeutisch verträglichen Hilfsstoff, ausgewählt aus der Gruppe bestehend aus einem Bindemittel, einem Sprengmittel und einem Benetzungsmittel.

2. Feste orale Dosierungsform mit sofortiger Freisetzung nach Anspruch 1, wobei das pharmazeutisch verträgliche Salz von AP1189 (*E*)-*N*-[1-(2-Nitrophenyl)-1-*H*-pyrrol-2-yl-allylidenamino]-guanidiniumacetat ist, einschließlich tautomerer und stereoisomerer Formen davon (AP1189-Acetat).

3. Feste orale Dosierungsform mit sofortiger Freisetzung nach Anspruch 1, wobei das pharmazeutisch verträgliche Salz von AP1189 (*E*)-*N*-[1-(2-Nitrophenyl)-1-*H*-pyrrol-2-yl-allylidenamino]-guanidiniumsuccinat ist, einschließlich tautomerer und stereoisomerer Formen davon (AP1189-Succinat).

4. Feste orale Dosierungsform mit sofortiger Freisetzung nach einem der Ansprüche 1 bis 3, wobei die feste orale Dosierungsform eine Tablette mit sofortiger Freisetzung ist.

5. Feste orale Dosierungsform mit sofortiger Freisetzung nach einem der Ansprüche 1 bis 4, wobei sich nicht weniger als 80 % des Salzes in 15 Minuten bei einem pH-Wert von 1 bis 3 unter Verwendung eines USP-2-Paddelgeräts bei 37 °C bei einer Rotationsgeschwindigkeit von 50 U/min in wässriger Lösung auflösen, wobei das Auflösungsmedium aus 500 ml 0,1 N HCl + 50 ml Wasser besteht, das nach Zugabe der Tablette in die Auflösungskammer hinzugefügt wird.

6. Feste orale Dosierungsform mit sofortiger Freisetzung nach einem der Ansprüche 1 bis 5, wobei sich nicht weniger als 80 % des Salzes in 15 Minuten bei einem pH-Wert von 1,2 unter Verwendung eines USP-2-Paddelgeräts bei 37 °C bei einer Rotationsgeschwindigkeit von 50 U/min in wässriger Lösung auflösen, wobei das Auflösungsmedium aus 500 ml 0,1 N HCl + 50 ml Wasser besteht, das nach Zugabe der Tablette in die Auflösungskammer hinzugefügt wird.

7. Feste orale Dosierungsform mit sofortiger Freisetzung nach einem der Ansprüche 1 bis 6, wobei die feste orale Dosierungsform das pharmazeutisch verträgliche Salz von AP1189 in einer Menge von 25 mg bis 250 mg (berechnet als die freie Base) umfasst; und wobei sich nicht weniger als 80 % des Salzes in 15 Minuten bei einem pH-Wert von 1 bis 3 unter Verwendung eines USP-2-Paddelgeräts bei 37 °C bei einer Rotationsgeschwindigkeit von 50 U/min in wässriger Lösung auflösen, wobei das Auflösungsmedium aus 500 ml 0,1 N HCl + 50 ml Wasser besteht, das nach Zugabe der Tablette in die Auflösungskammer hinzugefügt wird.

8. Feste orale Dosierungsform mit sofortiger Freisetzung nach einem der Ansprüche 1 bis 7, wobei die Cₘₐₓ der festen oralen Dosierungsform nach Verabreichung einer Einzeldosis des pharmazeutisch verträglichen Salzes von AP1189 nicht weniger als 180 ng/ml, wie nicht weniger als 200 ng/ml, wie nicht weniger als 225 ng/ml, wie nicht weniger als 250 ng/ml, wie nicht weniger als 275 ng/ml, wie nicht weniger als 300 ng/ml, wie nicht weniger als 350 ng/ml, wie nicht weniger als 400 ng/ml, wie nicht weniger als 450 ng/ml, wie nicht weniger als 500 ng/ml, wie nicht weniger als 600 ng/ml, wie nicht weniger als 700 ng/ml, wie nicht weniger als 800 ng/ml, wie nicht weniger als 900 ng/ml, wie nicht weniger als 1000 ng/ml, wie nicht weniger als 1200 ng/ml, wie nicht weniger als 1400 ng/ml, wie nicht weniger als 1600 ng/ml, wie nicht weniger als 1800 ng/ml, wie nicht weniger als 2000 ng/ml, wie nicht weniger als 2200 ng/ml, wie nicht weniger als 2400 ng/ml, wie nicht weniger als 2600 ng/ml, wie nicht weniger als 2800 ng/ml, wie nicht weniger als 3000 ng/ml, wie nicht weniger als 3200 ng/ml, wie nicht weniger als 3400 ng/ml, wie nicht weniger als 3600 ng/ml beträgt.

9. Feste orale Dosierungsform mit sofortiger Freisetzung nach einem der Ansprüche 1 bis 8, wobei das pharmazeutisch verträgliche Salz von AP1189 in einer Dosierung von 25 mg bis 650 mg AP1189 pro Dosierungsform vorhanden ist, wie 25 mg, wie 50 mg, wie 100 mg, wie 150 mg, wie 200 mg, wie 250 mg, wie 300 mg, wie 350 mg, wie 400 mg, wie 450 mg, wie 500 mg, wie 550 mg, wie 600 mg, wie 650 mg des pharmazeutisch verträglichen Salzes von AP1189, berechnet als die freie Base.

10. Feste orale Dosierungsform mit sofortiger Freisetzung nach einem der Ansprüche 1 bis 9, wobei das pharmazeutisch verträgliche Salz von AP1189 eine polymorphe Form von (*E*)-*N*-[1-(2-Nitrophenyl)-1-*H*-pyrrol-2-yl-allylidenamino]-guanidiniumacetat, einschließlich tautomerer und stereoisomerer Formen davon (AP1189-Acetat), oder eine polymorphe Form von (*E*)-*N*-[1-(2-Nitrophenyl)-1-*H*-pyrrol-2-yl-allylidenamino]-guanidiniumsuccinat ist, einschließlich tautomerer und stereoisomerer Formen davon (AP1189-Succinat).

11. Feste orale Dosierungsform mit sofortiger Freisetzung nach einem der vorhergehenden Ansprüche zur Verwendung beim Behandeln oder Vorbeugen einer Erkrankung, ausgewählt aus einer Nierenerkrankung, einer arthritischen Erkrankung, wie rheumatoider Arthritis, einer entzündlichen viralen Erkrankung oder Störung, wie einer viralen Atemwegserkrankung oder - störung, einer kardiovaskulären Erkrankung, Atherosklerose und einer systemischen entzündlichen Störung, wie einer Autoimmunerkrankung.

12. Feste orale Dosierungsform mit sofortiger Freisetzung nach einem der Ansprüche 1 bis 10 oder Verwendung nach Anspruch 11, wobei das pharmazeutisch verträgliche Salz von AP1189 in einer Menge von 1 mg bis 1000 mg verabreicht wird, wie 1 bis 5 mg, 5 bis 10 mg, 10 bis 15 mg, 15 bis 20 mg, 20 bis 25 mg, 25 bis 50 mg, 50 bis 75 mg, 75 bis 100 mg, 100 bis 125 mg, 125 bis 150 mg, 150 bis 175 mg, 175 bis 200 mg, 200 bis 250 mg, 250 bis 300 mg, 300 bis 350 mg, 350 bis 400 mg, 400 bis 450 mg, 450 bis 500 mg, 500 bis 600 mg, 600 bis 700 mg, 700 bis 800 mg, 800 bis 900 mg oder 900 bis 1000 mg des pharmazeutisch verträglichen Salzes von AP1189 pro Dosierung, berechnet als die freie Base.

13. Feste orale Dosierungsform mit sofortiger Freisetzung nach einem der Ansprüche 1 bis 10 oder die Verwendung nach den Ansprüchen 11 bis 12, wobei das pharmazeutisch verträgliche Salz von AP1189 einmal am Tag (QD), zweimal am Tag (BID) oder dreimal am Tag (TID) verabreicht wird.

14. Feste orale Dosierungsform mit sofortiger Freisetzung nach einem der Ansprüche 1 bis 10 oder die Verwendung nach den Ansprüchen 11 bis 12, wobei das pharmazeutisch verträgliche Salz von AP1189 in einer einmal täglichen Dosierung von 1 bis 1000 mg verabreicht wird.

## Revendications

1. Forme posologique orale solide à libération immédiate comprenant un sel pharmaceutiquement acceptable de l'AP1189 ((*E*)-*N*-[1-(2-nitrophényl)-1-*H*-pyrrole-2-yl-allylidèneamino]-guanidine) de formule I : 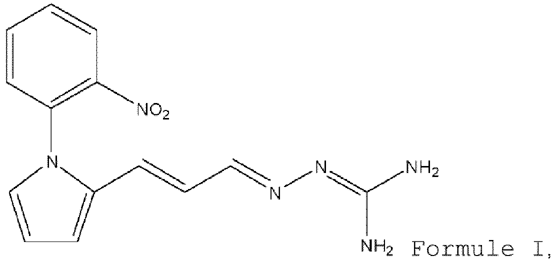
y compris ses formes tautomères et stéréoisomères,
dans laquelle ledit sel pharmaceutiquement acceptable est sélectionné parmi le groupe constitué de :
acétate de (*E*)-*N*-[1-(2-nitrophényl)-1-*H*-pyrrole-2-yl-allylidèneamino]-guanidinium, y compris ses formes tautomères et stéréoisomères ; et
succinate de (*E*)-*N*-[1-(2-nitrophényl)-1-*H*-pyrrole-2-yl-allylidèneamino]-guanidinium, y compris ses formes tautomères et stéréoisomères ;
et comprenant également au moins un excipient pharmaceutiquement acceptable sélectionné parmi le groupe constitué d'un liant, d'un désintégrant et d'un agent mouillant.

2. Forme posologique orale solide à libération immédiate selon la revendication 1, dans laquelle le sel pharmaceutiquement acceptable d'AP1189 est l'acétate de (*E*)-*N*-[1-(2-nitrophényl)-1-*H*-pyrrole-2-yl-allylidèneamino]-guanidinium, y compris ses formes tautomères et stéréoisomères (acétate d'AP1189).

3. Forme posologique orale solide à libération immédiate selon la revendication 1, dans laquelle le sel pharmaceutiquement acceptable d'AP1189 est le succinate de (*E*)-*N*-[1-(2-nitrophényl)-1-*H*-pyrrole-2-yl-allylidèneamino]-guanidinium, y compris ses formes tautomères et stéréoisomères (succinate d'AP1189).

4. Forme posologique orale solide à libération immédiate selon l'une quelconque des revendications 1 à 3, dans laquelle ladite forme posologique orale solide est un comprimé à libération immédiate.

5. Forme posologique orale solide à libération immédiate selon l'une quelconque des revendications 1 à 4, dans laquelle pas moins de 80 % du sel se dissout dans une solution aqueuse en 15 minutes à un pH de 1 à 3, en utilisant un équipement à palettes USP 2 à 37 °C à une vitesse de rotation de 50 tr/min, dans laquelle le milieu de dissolution est constitué de 500 ml de HCl 0,1 N + 50 ml d'eau, ajoutés à la chambre de dissolution après l'ajout du comprimé.

6. Forme posologique orale solide à libération immédiate selon l'une quelconque des revendications 1 à 5, dans laquelle pas moins de 80 % du sel se dissout dans une solution aqueuse en 15 minutes à un pH de 1,2, en utilisant un équipement à palettes USP 2 à 37 °C à une vitesse de rotation de 50 tr/min, dans laquelle le milieu de dissolution est constitué de 500 ml de HCl 0,1 N + 50 ml d'eau, ajoutés à la chambre de dissolution après l'ajout du comprimé.

7. Forme posologique orale solide à libération immédiate selon l'une quelconque des revendications 1 à 6, dans laquelle ladite forme posologique orale solide comprend ledit sel pharmaceutiquement acceptable d'AP1189 en une quantité comprise entre 25 mg à 250 mg (calculée en base libre) ; et dans laquelle pas moins de 80 % du sel se dissout dans une solution aqueuse en 15 minutes à un pH de 1 à 3, en utilisant un équipement à palettes USP 2 à 37 °C à une vitesse de rotation de 50 tr/min, dans laquelle le milieu de dissolution est constitué de 500 ml de HCl 0,1 N + 50 ml d'eau, ajoutés à la chambre de dissolution après l'ajout du comprimé.

8. Forme posologique solide orale à libération immédiate selon l'une quelconque des revendications 1 à 7, dans laquelle la Cₘₐₓ de la forme posologique solide orale n'est pas inférieure à 180 ng/mL, et notamment pas inférieure à 200 ng/mL, et notamment pas inférieure à 225 ng/mL, et notamment pas inférieure à 250 ng/mL, et notamment pas inférieure à 275 ng/mL, et notamment pas inférieure à 300 ng/mL, et notamment pas inférieure à 350 ng/mL, et notamment pas inférieure à 400 ng/mL, et notamment pas inférieure à 450 ng/mL, et notamment pas inférieure à 500 ng/mL,et notamment pas inférieure à 600 ng/mL, et notamment pas inférieure à 700 ng/mL, et notamment pas inférieure à 800 ng/mL, et notamment pas inférieure à 900 ng/mL, et notamment pas inférieure à 1000 ng/mL, et notamment pas inférieure à 1200 ng/mL, et notamment pas inférieure à 1400 ng/mL, et notamment pas inférieure à 1600 ng/mL, et notamment pas inférieure à 1800 ng/mL, et notamment pas inférieure à 2000 ng/mL, et notamment pas inférieure à 2200 ng/mL, et notamment pas inférieure à 2400 ng/mL, et notamment pas inférieure à 2600 ng/mL, et notamment pas inférieure à 2800 ng/mL, et notamment pas inférieure à 3000 ng/mL, et notamment pas inférieure à 3200 ng/mL, et notamment pas inférieure à 3400 ng/mL, et notamment pas inférieure à 3600 ng/mL après administration d'une dose unique dudit sel pharmaceutiquement acceptable d'AP1189.

9. Forme posologique orale solide à libération immédiate selon l'une quelconque des revendications 1 à 8, dans laquelle le sel pharmaceutiquement acceptable d'AP1189 est présent à une dose de 25 mg à 650 mg d'AP1189 par forme posologique, telle que 25 mg, telle que 50 mg, telle que 100 mg, telle que 150 mg, telle que 200 mg, telle que 250 mg, telle que 300 mg, telle que 350 mg, telle que 400 mg, telle que 450 mg, telle que 500 mg, telle que 550 mg, telle que 600 mg, telle que 650 mg du sel pharmaceutiquement acceptable d'AP1189 calculé en tant que base libre.

10. Forme posologique orale solide à libération immédiate selon l'une quelconque des revendications 1 à 9, dans laquelle le sel pharmaceutiquement acceptable d'AP1189 est une forme polymorphe de l'acétate de (*E*)-*N*-[1-(2-nitrophényl)-1-*H*-pyrrole-2-yl-allylidèneamino]-guanidinium, y compris ses formes tautomères et stéréoisomères (acétate d'AP1189), ou une forme polymorphe du succinate de (*E*)-*N*-[1-(2-nitrophényl)-1-*H*-pyrrole-2-yl-allylidèneamino]-guanidinium, y compris ses formes tautomères et stéréoisomères (succinate d'AP1189).

11. Forme posologique orale solide à libération immédiate selon l'une quelconque des revendications précédentes, destinée à être utilisée dans le traitement ou la prévention d'une maladie sélectionnée parmi une maladie rénale, une maladie arthritique telle que la polyarthrite rhumatoïde, une maladie ou un trouble viral inflammatoire tel qu'une maladie ou un trouble respiratoire viral, une maladie cardiovasculaire, l'athérosclérose et un trouble inflammatoire systémique tel qu'un trouble auto-immun.

12. Forme posologique orale solide à libération immédiate selon l'une quelconque des revendications 1 à 10, ou utilisation selon la revendication 11, dans laquelle ledit sel pharmaceutiquement acceptable d'AP1189 est administré en une quantité de 1 mg à 1000 mg, par exemple 1 à 5 mg, 5 à 10 mg, 10 à 15 mg, 15 à 20 mg, 20 à 25 mg, 25 à 50 mg, 50 à 75 mg, 75 à 100 mg, 100 à 125 mg, 125 à 150 mg, 150 à 175 mg, 175 à 200 mg, 200 à 250 mg, 250 à 300 mg, 300 à 350 mg, 350 à 400 mg, 400 à 450 mg, 450 à 500 mg, 500 à 600 mg, 600 à 700 mg, 700 à 800 mg, 800 à 900 mg ou 900 à 1000 mg du sel pharmaceutiquement acceptable d'AP1189, par dose calculée comme base libre.

13. Forme posologique orale solide à libération immédiate selon l'une quelconque des revendications 1 à 10, ou utilisation selon les revendications 11 et 12, dans laquelle ledit sel pharmaceutiquement acceptable d'AP1189 est administré une fois par jour (QD), deux fois par jour (BID) ou trois fois par jour (TID).

14. Forme posologique orale solide à libération immédiate selon l'une quelconque des revendications 1 à 10, ou utilisation selon les revendications 11 et 12, dans laquelle ledit sel pharmaceutiquement acceptable d'AP1189 est administré à une dose quotidienne unique de 1 à 1000 mg.
